(19) Europäisches Patentamt
European Patent Office
Office européen des brevets

(11) Publication number: 0 276 101 A2

(12) EUROPEAN PATENT APPLICATION

(21) Application number: 88300343.6

(22) Date of filing: 15.01.88

(51) Int. Cl.⁴: C 07 K 5/00
A 61 K 37/64

(30) Priority: 19.01.87 GB 8701076
19.01.87 GB 8701077
19.01.87 GB 8701078

(43) Date of publication of application:
27.07.88 Bulletin 88/30

(84) Designated Contracting States:
BE CH DE ES FR GB IT LI NL

(71) Applicant: ICI AMERICAS INC
Concord Pike & New Murphy Road
Wilmington Delaware 19897 (US)

(72) Inventor: Lewis, Joseph James
1623 Linda Drive
West Chester Pennsylvania 19380 (US)

Perkins, Charles William
1303 Delaware Avenue Apartment 1102
Wilmington Delaware 19806 (US)

Trainor, Diane Amy
7 Marshall Road
Glen Mills Pennsylvania 19342 (US)

Wildonger, Richard Alan
51 Morgan Hollow Way
Landenberg Pennsylvania 18350 (US)

(74) Representative: Smith, Stephen Collyer et al
Imperial Chemical Industries PLC Legal Department:
Patents Po Box 6 Bessemer Road
Welwyn Garden City Herts, AL7 1HD (GB)

# (54) Substituted peptide derivatives.

(57) The invention provides a series of novel substituted peptide derivatives of the formulae Ia, Ib and Ic (set out hereinafter) and pharmaceutically acceptable salts thereof, in which the radicals A, $R^1$, $R^2$ and $R^4$-$R^9$ have the meanings defined in the following specification. The compounds of formulae Ia, Ib, and Ic are inhbitors of human leukocytic elastase. The invention also provides pharmaceutical compositions containing a compound of formula Ia, Ib or Ic, or a pharmaceutically acceptable salt thereof and processes and intermediates for the manufacture of compounds of formulae Ia, Ib, and Ic.

EP 0 276 101 A2

## Description

### SUBSTITUTED PEPTIDE DERIVATIVES

Simple peptidyl fluoromethyl ketones, including trifluoromethyl ketones, are described as potential serine portease inhibitors in B. Imperiali and R. H. Abeles, Tetrahedron Lett., 27(2), 135-8 (1986). The present invention relates to certain substituted peptidyl trifluoromethyl ketone derivatives which are human leukocyte elastase (HLE) inhibitors making them useful whenever such inhibition is desired, such as for research tools in pharmacological, diagnostic and related studies, and in the treatment of tissue degenerative diseases such as pulmonary emphysema, atherosclerosis, rheumatoid arthritis and osteo arthritis in warm blooded animals. The invention also includes intermediates useful in the synthesis of these peptide derivatives, processes for preparing them, pharmaceutical compositions containing such peptide derivatives and methods for their use.

The substituted peptide derivatives of the present invention may be represented by the following formulae Ia, Ib and Ic:

(Formula set out on pages following Examples) Ia
(Formula set out on pages following Examples) Ib
(Formula set out on pages following Examples) Ic

wherein

$R^1$ is an alkyl group containing from 1 to 5 carbon atoms (for example, methyl, ethyl, n-propyl, iso-propyl, n-butyl, sec-butyl, iso-butyl, n-pentyl, pent-2-yl, pent-3-yl, 2-methylbutyl, and 3-methylbutyl), and more preferably from 2 to 5 carbons;

$R^2$ and $R^5$ are each selected independently from a group consisting of:

(I) an alkyl group containing from 1 to 10 carbons (for example, methyl, ethyl, n-propyl, iso-propyl, n-butyl, sec-butyl, iso-butyl, n-pentyl, pent-2-yl, pent-3-yl, 2-methylbutyl, 3-methylbutyl, hexyl, heptyl, octyl, nonyl, and decyl);

(II) an alkyl group containing from 1 to 6 carbon atoms (for example, methyl, ethyl, n-propyl, iso-propyl, n-butyl, sec-butyl, iso-butyl, n-pentyl, pent-2-yl, pent-3-yl, 2-methylbutyl, 3-methylbutyl, and hexyl) substituted by at least one member selected from a group consisting of:

(a) hydroxy;
(b) amino;
(c) alkylamino containing from 1 to 6 carbons;
(d) dialkylamino wherein each alkyl group contains from 1 to 6 carbons;
(e) alkanoyl containing from 1 to 6 carbons;
(f) arylcarbonyl wherein the aryl contains 6, 10 or 12 carbons;
(g) aralkanoyl containing 8 to 13 carbons;
(h) amido which may be attached to the alkyl group via either a nitrogen or carbon of said amido;
(i) alkylcarbonylamino wherein the alkyl group contains from 1 to 6 carbons;
(j) alkylaminocarbonyl wherein the alkyl group contains from 1 to 6 carbons;
(k) arylcarbonylamino wherein the aryl group contains 6, 10 or 12 carbons;
(l) aralkylcarbonylamino wherein the aralkyl group contains from 7 to 13 carbons;
(m) arylaminocarbonyl wherein the aryl group contains 6, 10 or 12 carbons;
(n) aralkylaminocarbonyl wherein the aralkyl group contains from 7 to 13 carbons;
(o) carboxy;
(p) aryloxycarbonyl wherein the aryl group contains 6, 10 or 12 carbons;
(q) aralkoxycarbonyl wherein the aralkoxy group contains from 7 to 13 carbons;
(r) alkanoyloxy containing from 1 to 6 carbons;
(s) aroyloxy wherein the aryl portion contains 6, 10 or 12 carbons;
(t) aralkanoyloxy containing from 8 to 14 carbons;
(u) alkylsulfonamido wherein the alkyl group contains from 1 to 6 carbons;
(v) aralkylsulfonamido wherein the aralkyl group contains from 7 to 13 carbons;
(w) arylsulfonamido wherein the aryl group contains 6, 10 or 12 carbons;
(x) acylsulfonamido (i.e., acylaminosulfonyl and sulfonylaminocarbonyl) (1 to 15 carbons) including acylsulfonamido wherein the acyl group contains 1 to 7 carbons when it is the terminal portion of the acylsulfonamide and provided that when the acylsulfonamido contains an aryl the aryl may be further substituted by a member selected from fluoro, chloro, bromo, iodo and nitro;
(y) alkoxycarbonyl wherein the alkoxy group contains from 1 to 6 carbons;
(z) aralkoxycarbonylamino containing from 8 to 13 carbons (e.g., benzyloxycarbonyl amino);
(aa) aryloxycarbonylamino wherein the aryloxy group contains 6, 10 or 12 carbons;
(bb) alkoxycarbonylamino wherein the alkyloxy group contains from 1 to 6 carbons;
(cc) aryl containing 6, 10 or 12 carbons (e.g., phenyl, biphenyl, naphthyl);
(ee) aryl containing 6, 10 or 12 carbons and substituted by 1 to 3 members selected from a group consisting of chloro, bromo, iodo, fluoro, trifluoromethyl, hydroxy, alkyl (1 to 6 carbons), alkoxy (1 to 6 carbons), alkoxycarbonyl (1 to 6 carbons), carboxy, 5-tetrazolo, and acylsufonamido (i.e., acylaminosulfonyl and sulfonylaminocarbonyl) (1 to 15 carbons) and provided that when the acylsulfonamido contains an aryl the aryl may be further substituted by a member selected from

fluoro, chloro, bromo, iodo and nitro;

(ee) cycloalkyl containing from 3 to 15 carbons (e.g., cyclohexyl, adamantyl, norbornyl);

(ff) alkylureido wherein the alkyl group contains from 1 to 6 carbons;

(gg) aralkylureido containing from 8 to 13 carbons; and

(hh) arylureido wherein the aryl group contains 6, 10 or 12 carbons; and

(III) phenyl;

$R^4$ and $R^6$ are each independently hydrogen or methyl; and

A is an acidic radical selected from a group consisting of a carbamoylsulfonamido radical of formula $R^7.NH.CO.NH.S(O_2)$-, a sulfonylureido radical of formula $R^9.S(O_2).NH.CO.NR^8$-, a heterocyclic acylsulfonamido radical of formula Q.B-, and a trifluoromethylsulfonamido radical of formula $CF_3.S(O_2).NH$- wherein

$R^7$ is selected from a group consisting of:

(I) an alkyl group containing from 1 to 10 carbons (for example, as listed above for an alkyl group of $R^2$ or $R^5$);

(II) a cycloalkyl group containing from 3 to 10 carbons (for example, cyclopropyl, cyclobutyl, cyclopentyl, cyclohexyl, norbornyl, and adamantyl);

(III) an arylmethyl group containing 7 to 11 carbons (for example, benzyl and naphthylmethyl) wherein the aryl group is optionally substituted by 1 to 3 members of the group consisting of fluoro, chloro, bromo, iodo, trifluoromethyl, nitro, an alkyl group of 1 to 4 carbons, methoxy, ethoxy, and methylthio;

(IV) an aryl group containing 6 or 10 carbons (for example, phenyl or naphthyl) and optionally substituted by 1 to 3 members of the group consisting of fluoro, chloro, bromo, iodo, trifluoromethyl, nitro, an alkyl group of 1 to 4 carbons, methoxy, ethoxy, and methylthio;

(V) an aromatic heterocyclic group as defined below for Q (for example, pyridyl, thienyl, furyl and quinolinyl);

$R^8$ is hydrogen or methyl;

$R^9$ is selected from a group consisting of:

(I) an alkyl group containing from 1 to 10 carbons (for example, as listed above for an alkyl group of $R^2$ or $R^5$);

(II) a cycloalkyl group containing from 3 to 10 carbons (for example, cyclopropyl, cyclobutyl, cyclopentyl, cyclohexyl, norbornyl, and adamantyl);

(III) an aryl group containing 6 or 10 carbons (for example, phenyl or naphthyl) and optionally substituted by 1 to 3 members of the group consisting of fluoro, chloro, bromo, iodo, trifluormethyl, methyl, nitro, hydroxy, carboxy, amino, dimethylamino, phenyl, and an alkylcarbonylamino group wherein the alkyl group contains 1 to 4 carbons;

(IV) an aromatic heterocyclic group as defined below for Q (for example, pyridyl, thienyl, furyl, and quinolinyl, such as, for example, 8-quinolinyl);

(V) a 2-phenylethyl group; and

(VI) a 2,2,2-trifluoroethyl group;

Q is an aromatic heterocyclic group of from 1 to 15 carbons and from 1 to 4 heteroatoms each of which is selected independently from a group consisting of sulfur, nitrogen and oxygen, and which form 1 to 3 five or six-membered rings at least one of which is aromatic, and optionally, wherein up to 3 carbons of the aromatic ring(s) may be substituted at any carbon atom with a member of a group consisting of fluoro, chloro, bromo, iodo and trifluoromethyl, and provided further that any nitrogen may be substituted by an alkyl group containing from 1 to 6 carbons (for example, pyridyl, thienyl, furyl, and quinolinyl, such as, for example, 2-pyridyl); and

B is an acylsulfonamide linkage selected from a group consisting of those represented by formulae (i) $-S(O_2)NHCO-$ and (ii) $-CONHS(O_2)-$; and

where appropriate, the acid- and base-addition salts thereof.

Compounds of formulae Ia, Ib and Ic are referred to herein as dipeptides, tripeptides and tetrapeptides, respectively.

Particular values for compounds of the invention include the following members of the groups defined above:

A is selected from the group defined above for A;

$R^1$ is an alkyl group containing 3 carbons;

$R^2$ and $R^5$ (when present) are each selected independently from a group consisting of:

(I) an alkyl group containing from 1 to 4 carbons;

(II) an alkyl group containing from 1 to 4 carbons substituted by at least one member selected from a group consisting of:

(e) alkanoyl containing from 1 to 3 carbons;

(f) arylcarbonyl wherein the aryl contains 6 or 10 carbons (e.g., phenyl or naphthyl);

(g) aralkanoyl containing 8 carbons (e.g., phenylacetyl);

(h) amido which may be attached to the alkyl group via either a nitrogen or carbon of said amido;

(i) alkylcarbonylamino wherein the alkyl group contains from 1 to 2 carbons;

(j) alkylaminocarbonyl wherein the alkyl group contains from 1 to 2 carbons;

(k) arylcarbonylamino wherein the aryl group contains 6 carbons (e.g., phenyl);

(l) aralkylcarbonylamino wherein the aralkyl group contains 7 carbons;

(m) arylaminocarbonyl wherein the aryl group contains 6 carbons;
(n) aralkyaminocarbonyl wherein the aralkyl group contains 7 carbons;
(o) carboxy;
(p) aryloxycarbonyl wherein the aryl group contains 6 carbons;
(q) aralkoxycarbonyl wherein the aralkoxy group contains 7 carbons;
(r) alkanoyloxy containing from 1 to 2 carbons;
(s) aroyloxy wherein the aryl portion contains 6 carbons;
(t) aralkanoyloxy containing 8 carbons;
(u) alkylsulfonamido wherein the alkyl group contains from 1 to 6 carbons;
(v) aralkylsulfonamido wherein the aralkyl group contains from 7 to 13 carbons (e.g., 1-naphthylmethylsulfonylamino or 4-phenylbutylsulfonylamino);
(w) arylsulfonamido wherein the aryl group contains 6 or 10 carbons;
(x) acylsulfonamido containing 1 to 15 carbons (e.g. phenylsulfonylaminocarbonyl);
(y) alkoxycarbonyl wherein the alkoxy group contains from 1 to 2 carbons;
(z) aralkoxycarbonylamino wherein the aralkoxy group contains 7 carbons (e.g., benzyloxycarbonylamino);
(aa) aryloxycarbonylamino wherein the aryloxy group contains 6 carbons;
(bb) alkoxycarbonylamino wherein the alkyloxy group contains from 1 to 3 carbons;
(cc) aryl containing 6 or 10 carbons (e.g., phenyl or naphthyl);
(dd) aryl containing 6 or 10 carbons and substituted by 1 to 3 members selected from a group consisting of chloro, bromo, iodo, fluoro, trifluoromethyl, hydroxy, alkyl (1 to 2 carbons), alkoxy (1 to 2 carbons), alkoxy carbonyl (2 to 3 carbons), carboxy, 5-tetrazolo and acylsufonamido (1 to 15 carbons);
(ee) cycloalkyl containing from 3 to 15 carbons (e.g., cyclohexyl, adamantyl, norbornyl);
(ff) alkylureido wherein the alkyl group contains from 1 to 2 carbons;
(gg) aralkylureido wherein the aralkyl group contains 7 carbons; and
(hh) arylureido wherein the aryl group contains 6 or 10 carbons; and
(III) phenyl;

$R^4$, $R^6$ and $R^8$ (when present) are each hydrogen; and
$R^7$, $R^9$, Q and B (when present) are selected from the groups defined above for $R^7$, $R^9$, Q and B, respectively.

More particular values include:
A selected from the group defined above for A;
$R^1$ selected to be isopropyl;
$R^2$ (when present) selected from a group consisting of:
(I) an alkyl group containing 2 to 3 carbons;
(II)(q) ethyl substituted by aralkoxycarbonyl wherein the aralkoxy group contains 7 carbons;
(w) butyl substituted by an arylsulfonamido wherein the aryl portion has 6 carbons;
(x) ethyl substituted by acylsulfonamido containing 7 carbons (e.g., 2-(phenylsulfonylaminocarbonyl)ethyl);
(z) butyl substituted by aralkyloxycarbonylamino wherein the aralkoxy portion contains 7 carbons, (e.g., benzyloxycarbonylamino); and
(cc) methyl substituted by an aryl containing 6 carbons; and
(III) phenyl;
$R^4$, $R^6$ and $R^8$ (when present) each selected as hydrogen; and
$R^5$ (when present) selected from a group consisting of: (I) n-butyl;
(II)(q) ethyl substituted by aralkoxycarbonyl wherein the aralkoxy contains 7 carbons; and
(II)(z) butyl substituted by aralkyloxycarbonylamino wherein the aralkyl group contains 7 carbons; and
$R^7$, $R^9$, Q and B (when present) selected from the groups defined above for $R^7$, $R^9$, Q and B, respectively.

The following provisos apply to the compounds of this invention:
(1) alkyls may be straight or branched chain; and
(2) no carbon of an alkyl may be directly bonded to two heteroatoms.

The salts of the compounds of formulae Ia, Ib and Ic include pharmaceutically-acceptable base- or acid-addition salts such as those made with a mineral acid, e.g., hydrochloric, or an organic acid such as citric, maleic, fumaric or acetic. Base-addition salts include those made with alkali metal hydroxides such as sodium hydroxide, alkali metal carbonates and bicarbonates, alkaline earth hydroxides and organic amine salts. Such salts may be prepared by dissolving the peptide derivative in a mixture of water and a water-miscible organic solvent, adding an aqueous solution of the base and recovering the salt from the aqueous solution.

The preferred compounds of the present invention are of the S configuration (i.e., that of the naturally occurring L-amino acids) at chiral centers identified by * in formulae IIa, IIb, and IIc below. The methods of synthesis described below generally provide a diastereomeric mixture as a result of the presence of the products with both the R and the S configurations at the chiral center identified by the symbol Δ, i.e., 3(RS) compounds. The methods of separation and synthesis described below for chiral synthesis provide compounds which are substantially enantiomerically and diastereomerically pure. The preferred compounds are those assigned the S configuration at the center identified by the symbol Δ, i.e. 3(S) compounds.

(Formula set out in pages following Examples) IIa
(Formula set out in pages following Examples) IIb

(Formula set out in pages following Examples) IIc

As will be appreciated by those skilled in the art, the activity of the individual isomers is not the same, and it is therefore preferred to utilize the more active isomer. The present invention includes both the diastereomeric mixture and the active S and R isomers.

As will be appreciated by those skilled in the art, the trifluoromethyl ketones can exist as solvates, particularly hydrates, represented by formulae IIIa, IIIb and IIIc, and these are encompassed by the present invention.

(Formula set out in pages following Examples) IIIa
(Formula set out in pages following Examples) IIIb
(Formula set out in pages following Examples) IIIc

It is preferred to prepare the peptide derivatives of the present invention from commercially available alpha amino acids (i.e., those in which the $NH_2$ group is attached to the carbon atom next to the -COOH group). Because of this the preferred $R^2$ and $R^5$ substitutents in the above formulae for tri- and tetrapeptide derivatives are those obtained from one of the following amino acids: alanine, valine, norvaline, leucine, isoleucine, norleucine, phenylalanine, tryptophan, tyrosine, asparagine, glutamine, aspartic acid, glutamic acid, lysine, threonine, serine, α-aminobutyric acid, and phenylglycine, as appropriate.

Preferred groups of compounds include compounds of formulae Ia, Ib, and Ic in which the values for A, $R^1$, $R^2$, $R^4$, $R^5$, $R^6$, $R^7$, $R^8$, $R^9$, Q and B are selected from the values defined above as particular values.

More preferred groups of compounds include compounds of formulae Ia, Ib, and Ic in which the values for A, $R^1$, $R^2$, $R^4$, $R^5$, $R^6$, $R^7$, $R^8$, $R^9$, Q and B are selected from the values defined above as more particular values.

It is especially preferred that the radical A be in the para position to the carbonyl group.

A group of special interest is one represented by formula Ib, wherein $R^1$ and $R^2$ are isopropyl, $R^4$ is hydrogen and the radical A is para to the carbonyl group.

Subgroups of the above described groups include one wherein A is a carbamoylsulfonamido radical of formula $R^7$.NH.CO.NH.S($O_2$)-, one wherein A is a sulfonylureido radical of formula $R^9$.S($O_2$).NH.CO.$NR^8$-, one wherein A is a heterocyclic acylsulfonamido radical of formula Q.B-, and one wherein A is a trifluoromethylsulfonamido radical of formula $CF_3$.S($O_2$).NH-.

Preferred values for groups contained in radicals of formula A in the above groups and subgroups include, when present:
for $R^7$: an optionally substituted aryl group, especially phenyl, 4-chlorophenyl and 1-naphthyl;
for $R^8$: hydrogen;
for $R^9$: an optionally substituted aryl group, especially phenyl and 4-chlorophenyl;
for Q: an optionally substituted pyridyl group, especially 2-pyridyl; and
for B: a linkage of formula -S($O_2$).NH.CO-.

Particularly preferred compounds of the invention are 3(RS)-[4-[(N′-phenylureido)sulfonyl]-benzoyl]-L-valyl-N-[3-(1,1,1-trifluoro-4-methyl-2-oxopentyl)]-L-prolinamide, 3(RS)-[4-[[N′-(4-chlorophenyl)ureido]sulfonyl]benzoyl]-L-valyl-N-[3-(1,1,1-trifluoro-4-methyl-2-oxopent-yl)]-L-prolinamide, 3(RS)-[4-[[N′-(1-naphthalenyl)ureido]sulfonyl]benzoyl]-L-valyl-N-[3-(1,1,1-trifluoro-4-methyl-2-oxopentyl)]-L-prolinamide, 3(RS)-[4-[N′-(phenylsulfonyl)-ureido]benzoyl]-L-valyl-N-[3-(1,1,1-trifluoro-4-methyl-2-oxopentyl)]-L-prolinamide, 3(RS)-[4-[N′-[(4-chlorophenyl)sulfonyl]ureido]benzoyl]-L-valyl-N-[3-(1,1,1-trifluoro-4-methyl-2-oxopentyl)]-L-prolinamide, 3(RS)-[4-[(2-pyridylsulfonylamino)carbonyl]benzoyl-L-valyl-N-[3-(1,1,1-trifluoro-4-methyl-2-oxopentyl)]-L-prolinamide, and 3(RS)-[4-[(trifluoromethylsulfonyl)amino]benzoyl]-L-valyl-N-[3-(1,1,1-trifluoro-4-methyl-2-oxopentyl)]-L-prolinamide.

According to a further feature of the invention there are provided pharmaceutical compositions comprising a pharmaceutically-effective amount of at least one peptide derivative of formula Ia, Ib or Ic and a pharmaceutically-acceptable diluent or carrier.

The compounds of formulae Ia, Ib and Ic may be made by processes which include processes known in the chemical art for the production of structurally analogous heterocyclic and peptidic compounds. Such processes for the manufacture of a compound of formula Ia, Ib or Ic as defined above are provided as further features of the invention and are illustrated by the following procedures in which the meanings of generic radicals are as defined above and M has the values defined below. Novel intermediates for the preparation of compounds of formulas Ia, Ib and Ic provide yet another aspect of the invention.

Conveniently, amino alcohols of formulae IVa, IVb and IVc:

(Formula set out in pages following Examples) IVa
(Formula set out in pages following Examples) IVb
(Formula set out in pages following Examples) IVc

are key intermediates in the preparation of compounds of formula Ia, Ib, and Ic, respectively. These amino alcohols may be prepared from an amino alcohol of formula V:

(Formula set out in pages following Examples) V using methods commonly known to those skilled in the art, such as those described in M. Bodanszky, Principles of Peptide Synthesis, Springer-Verlag, Berlin, (1984), and The Peptides. Analysis, Synthesis and Biology (ed. E. Gross and J. Meinhofer), Vols. 1-5, (Academic Press, New York) 1979-1983. Use of appropriately N-protected proline (such as CBZ-proline), followed by peptide coupling procedures and appropriate amino deprotection affords compounds of formula IVa. Similar coupling with appropriate N-protected dipeptide acids and tripeptide acids and deprotection affords compounds of formulae IVb and IVc, respectively. In addition, compounds of formula IVa can be converted into compounds of

IVb or IVc by utilizing the same peptide methodology.

An amino alcohol of formula V may be obtained by the reduction of the nitro group of a corresponding nitroalcohol of formula VI.

(Formula set out in pages following Examples) VI Henry condensation (McBee, E.T., et al, J. Amer. Chem. Soc., 78 4053 (1956)) of an appropriate nitroalkane of formula $R^1CH_2NO_2$ (prepared by standard methods if not otherwise available) with trifluoroacetaldehyde ethyl hemiacetal of formula $[CF_3CH(OH)OCH_2CH_3]$ provides a corresponding nitroalcohol of formula VI which is obtained as a mixture of two racemic diastereomers ([2(RS), 3(RS)] and [2(RS),3(SR)]). As is illustrated in Example 1, in which $R^1$ is isopropyl, it is preferably to affect a separation of the mixture of racemic diastereomers of formula VI by fractional distillation and crystallization to provide nitroalcohol VI as a substantially pure racemic diastereomer [2(RS), 3(SR)] substantially free of the other racemic diastereomer [2(RS),3(RS)]. Reduction of the nitro group by the preferred method of hydrogenation over 10% palladium on carbon catalyst provides a compound of formula V, conveniently isolated as the hydrochloride salt, as one racemic diastereomer [2(RS),3(SR)] substantially free of other racemic diastereomer. (It will be appreciated by one skilled in the art that, alternatively, the diastereomer [2(RS),3(RS)] may also be used for the production of compounds of this invention). This amine salt may be used directly for further synthesis.

As is further illustrated in Example 2, for preparation of a compound of formula IVb in which $R^1$ and $R^2$ are isopropyl and $R^4$ is hydrogen, the aminoalcohols of formulae IVa, IVb and IVc obtained by this method are substantially pure [2(RS),3(SR)] mixtures unless a separation is carried out.

The key intermediates of formula IVa, IVb and IVc may then be converted into corresponding compounds of formulae Ia, Ib and Ic by a variety of methods:

(A) Generally a compound of formula Ia, Ib or Ic may be prepared by oxidizing a corresponding hydroxy compound of formula VIIa, VIIb or VIIc:

(Formula set out in pages following Examples) VIIa
(Formula set out in pages following Examples) VIIb
(Formula set out in pages following Examples) VIIc

However, if a compound of formulae VIIa, VIIb or VIIc, contains a basic nitrogen, it is generally preferable to use an alternative method or to protect the basic nitrogen before oxidization and deprotect it after oxidation to provide the corresponding compound of formula Ia, Ib or Ic. Methods which are useful for oxidation include the use of oxalyl chloride, DMSO and a tertiary amine (see Marx, M. et al., J. Org. Chem., 49, 788-793 (1984) with the best results being obtained with 10-20 equivalents of oxidizing agent), the use of acetic anhydride and DMSO, the use of chromium trioxide pyridine complex in methylene chloride, and the use of Dess-Martin periodinane [1,1,1-triacetoxy-2,1-benzoxiodol-3(3H)-one] (method of Dess, D.B. et al, J. Org. Chem.,. 48, 4155-56 (1983)). The preferred method is the use of Dess-Martin periodinane. Unless they have been separated, the products Ia, Ib and Ic obtained by this method will contain a mixture which will consist substantially of two diastereomers [3(RS)] if the centers corresponding to those indicated with an * in formulae IIa, IIb, and IIc, respectively, are substantially enantiomerically pure, for example, as described in Examples 6, 7 and 10.

Compounds of formulae VIIa, VIIb and VIIc, respectively, may be obtained from the corresponding key intermediates of formula IVa, IVb and IVc by coupling them with an appropriate substituted benzoic acid of formula VIII:

(Formula set out in pages following Examples) VIII wherein M is A, using, for example, a water soluble carbodiimide, such as 1-ethyl-3-(dimethylaminopropyl)carbodiimide hydrochloride, and 4-dimethylaminopyridine in an organic solvent, such as, for example, a mixture of dimethylformamide and dichloromethane, at, for example, room temperature. Intermediate benzoic acids of formula VIII wherein M is A may be prepared by methods analogous to those described in the examples; by methods analogous to those described below and in the examples for preparation of intermediate compounds of formulae IXa, IXb and IXc and intermediate compounds of formula Xa, Xb and Xc; or by other, conventional means.

Alternatively, as described below, the intermediates of formulae IVa, IVb and IVc may be converted into corresponding intermediates of formulae IXa, IXb and IXc (wherein M is not A):

(Formula set out in pages following Examples) IXa
(Formula set out in pages following Examples) IXb
(Formula set out in pages following Examples) IXc

by a similar coupling with an acid of formula VIII (wherein M is not A): next, alcohols of formulae IXa, IXb and IXc may be converted into corresponding ketones of formula Xa, Xb and Xc (wherein M is not A):

(Formula set out in pages following Examples) Xa
(Formula set out in pages following Examples) Xb
(Formula set out in pages following Examples) Xc

using an oxidation method similar to one described above in (A); and, finally, the intermediate ketones of formulae Xa, Xb and Xc (wherein M is not A) may be elaborated to provide the corresponding products of formulae Ia, Ib and Ic.

(B) A compound of formula Ia, Ib or Ic wherein A is a radical of formula $R^7.NH.CO.NH.S(O_2)$-may be prepared by treating a corresponding compound of formula Xa, Xb or Xc wherein M is an aminosulfonyl radical of formula $H_2NS(O_2)$- with an isocyanate of formula $R^7.NCO$. For example, intermediates of formulae Xa, Xb and Xc wherein M is $H_2NS(O_2)$- may be treated with phenyl isocyanate in aqueous acetone containing sodium

hydroxide at about 10°C to provide the corresponding products of formulae Ia, Ib and Ic, wherein A is $R^7$.NH.CO.NH.S($O_2$)- in which $R^7$ is phenyl (see S. Peterson, Berichte Deutsche Chem. Gesel. zu Berlin, 83, 551 (1950)). Unless a separation is carried out, the products of formulae Ia, Ib and Ic, wherein A is $R^7$.NH.CO.NH.S($O_2$)- obtained by this method will be diasteriomeric mixtures [3(RS)] corresponding to the mixtures of the intermediates of formulae Xa, Xb and Xc wherein M is $H_2NS(O_2)$-from which they are prepared, for example, as described in Examples 3, 4 and 5.

Intermediates of formulae Xa, Xb and Xc wherein M is $H_2NS(O_2)$- may be obtained from corresponding intermediates of formulae IXa, IXb and IXc wherein M is $H_2NS(O_2)$- by using an oxidation method similar to one described in (A), for example as described in Example 3b. Intermediates of formulae IXa, IXb and IXc wherein M is $H_2NS(O_2)$- may be prepared from corresponding intermediates for formulae IVa, IVb and IVc by coupling them with an acid of formula VIII wherein M is $H_2NS(O_2)$-, for example, as described in Example 3a. If not commercially avail able, isocyanates of formula $R^7$.NCO may be prepared by conventional methods.

(C) A compound of formula Ia, Ib or Ic wherein A is a radical of formula $R^9$.S($O_2$).NH.CO.N$R^8$-may be prepared by treating a corresponding compound of formula Xa, Xb or Xc wherein M is an amino radical of formula HN$R^8$- with a sulfonylisocyanate of formula $R^9$.S($O_2$).NCO using an analogous method to that described in Example 6a for preparation of a benzoic acid of formula VIII wherein M is $R^9$S($O_2$).NH.CO.N$R^8$-, in which $R^8$ is hydrogen and $R^9$ is phenyl. Unless a separation is carried out, the products of formulae Ia, Ib, and Ic, wherein A is $R^9$S($O_2$).NH.CO.N$R^8$-obtained by this method will be diastereometric mixture [3(RS)] corresponding to the mixture of the intermediates of formulae Xa, Xb, and Xc wherein M is HN$R^8$- from which they are prepared.

Intermediates of formulae Xa, Xb, and Xc in which M is HN$R^8$- may be prepared by a variety of methods. An acid of formula VIII wherein M is an amino group of formula HN$R^8$- in which the nitrogen is further protected with, for example, a carbobenzoxy group, may be coupled with an amino acid alcohol of formula IVa, IVb or IVc to afford a corresponding alcohol of formula IXa, IXb or IXc, followed by oxidation by a similar method to one described in (A) and deprotection of the amino group, for example, by hydrogenolysis, to provide a corresponding compound of formula Xa, Xb or Xc wherein M is HN$R^8$-. Alternatively, an amine alcohol of formula IVa, IVb or IVc may be coupled with (or acylated by) an acid of formula VIII (or a reactive derivative thereof) wherein M is $R^a$OOC- wherein $R^a$ is a conveniently removed acid protecting group, for example an alkyl group of one to six carbons or a benzyl group, to provide a corresponding compound of formula IXa, IXb or IXc. For example, coupling an alcohol of formula IVa, IVb and IVc with a benzoic acid bearing a carbomethoxy substituent using a water soluble carbodiimide and conditions similar to those described in Example 8 provides a corresponding alcohol of formula IXa, IXb or IXc wherein M is $R^a$OOC- and $R^a$ is methyl. Oxidation of ester intermediates of formulae IXa, IXb, and IXc wherein M is $R^a$OOC- using a similar method to one described in (A) provides corresponding ester intermediates of formulae Xa, Xb, and Xc wherein M has the value COO$R^a$. A preferred method is the use of Dess-Martin periodinane. Conversion of ester intermediates of formulae Xa, Xb, and Xc wherein M has the value COO$R^a$ into the corresponding products of formula Xa, Xb, and Xc wherein M is HN$R^8$- and $R^8$ is hydrogen may be carried out by any of several possible sequences. One sequence involves decomposing esters of formulae Xa, Xb, and Xc wherein M has the value COO$R^a$ into the corresponding acids of formulae Xa, Xb, and Xc wherein M has the value COOH. By use of a modified Curtis reaction using, for example, diphenylphosphorylazide and triethylamine in benzene or toluene at 80° (see T. Shioiri, K. Ninomiya and S. Yamada, J. Amer. Chem. Soc., 94, 6203 (1972)), the acids of formulae Xa, Xb, and Xc wherein M has the value COOH may be converted into the corresponding isocyanates of formulae Xa, Xb, and Xc wherein M has the value NCO. By treatment, preferably in situ, with benzyl alcohol, the isocyanates of formulae Xa, Xb, and Xc wherein M has the value NCO may be converted into the corresponding carbamates of formulae Xa, Xb, and Xc wherein M has the value $NHCOOCH_2Ø$, which carbamates may be further converted by hydrogenolysis (for example by using hydrogen, a palladium on carbon catalyst, and ethanol as solvent at room temperature) into the corresponding amines of formulae Xa, Xb, and Xc wherein M has the value $NH_2$.

Sulfonylisocyanates of formula $R^9$.S($O_2$).NCO may be obtained from the corresponding sulfonamides of formula $R^9$.S($O_2$)$NH_2$ as described in U.S. Patent No. 4,394,506.

(D) Alternatively, a compound of formula Ia, Ib or Ic wherein A is a radical of formula $R^9$.S($O_2$).NH.CO.N$R^8$- wherein $R^8$ is H, may be prepared by treating a corresponding compound of formula Xa, Xb or Xc wherein M is an isocyanate radical of formula ONC- (prepared as described above in (C)) with a sulfonamide of formula $R^9$.S($O_2$).$NH_2$, for example in situ by using, for example, a base such as N-methylmorpholine and an added co-solvent such as dimethyl formamide at 50°, or by a modification of the Peterson method using, for example, sodium hydroxide in aqueous acetone at 0°. If not commercially available, sulfonamides of formula $R^9$.S($O_2$).$NH_2$ may be prepared by conventional methods.

(E) A compound of formula Ia, Ib or Ic wherein A is a radical of formula Q.B- in which -B-is -S($O_2$)NHCO- may be prepared by coupling a corresponding acid of formula Xa, Xb or Xc wherein M is COOH with a sulfonamide of formula Q.S($O_2$).$NH_2$. The coupling may be carried out using, for example, a water soluble carbodiimide, such as 1-ethyl-3-(3-dimethylaminopropyl)carbodiimide hydrochloride, and 4-dimethylaminopyridine in dichloromethane at room temperature, such as described in Example 9b. Acids of formulae Xa, Xb and Xc wherein M is COOH may be obtained as described above in (C). Heterocyclic sulfonamides of formula Q.S($O_2$).$NH_2$ may be prepared by conventional methods.

(F) A compound of formula Ia, Ib or Ic wherein A is a radical of formula Q.B- in which -B- is -CO.NH.S($O_2$)-

may be prepared by coupling a corresponding sulfonamide of formula Xa, Xb or Xc wherein M is $H_2N.S(O_2)$- with a heterocyclic acid of formula Q.COOH using a similar coupling method, for example, to that described above in (E). A sulfonamide of formula Xa, Xb or Xc wherein M is $H_2N.S(O_2)$- may be prepared as described above in (B). If not commercially available, a heterocyclic acid of formula Q.COOH may be prepared by a conventional method.

(G) A compound of formula Ia, Ib or Ic wherein A is a radical of formula $CF_3.S(O_2).NH$- may be prepared by treating a corresponding amine of formula Xa, Xb or Xc wherein M is $H_2N$- with trifluoromethanesulfonic anhydride, using, for example, a procedure analogous to the one described in Example 10a. An amine of formula Xa, Xb or Xc wherein M is $H_2N$- may be prepared as described above in (C).

Chiral Synthesis

As will be clear to one skilled in the art, if starting materials are used which are substantially diastereomerically pure at the centers corresponding to those indicated by * in formulae IIa, IIb, and IIc and these centers are not subsequently epimerized, products of formulae Ia, Ib, and Ic which are substantially enantiomerically and diastereomerically pure may be obtained by any procedure in which an intermediate containing the center indicated by Δ in formulae IIa, IIb and IIc is obtained in a substantially enantiomerically and diastereomerically pure form and in which the subsequent reactions do not result in an epimerization. In general, an anantiomerically and diastereomerically pure compound of formula Ia, Ib or Ic may be obtained by a separation of a corresponding compound of formula Ia, Ib or Ic which is a diastereomeric mixture (owing to the 3 (RS) center) (prepared, for example, by a method described above) into its single isomers which are substantially diastereomerically and enantiomerically pure. The preferred method for completing this separation is the use of preparative chromatography, e.g., medium pressure liquid chromatography or high pressure liquid chromatography.

It may be convenient to carry out a similar chromatographic separation of an intermediate of formula Xa, Xb or Xc, followed by conversion of the resulting substantially enantiomerically and diastereomerically pure intermediate of formula Xa, Xb or Xc into a compound of formula Ia, Ib or Ic by a method described above, using conditions which avoid epimerization.

Alternatively, an amine of formula V which is substantially enantiomerically and diastereomerically pure may be obtained by resolution, and the chiral synthesis completed using the resolved amine as an intermediate. For example, the free base of the racemic [2(RS),3(SR)] diastereomer of an amine of compound V may be resolved by formation of the diastereomeric salts with D-tartaric acid and separation of these salts, for example as described in Example 11. The salt containing the 2(R),3(S) isomer of the amine of compound V may then be used in a procedure analogous to those of Example 2 to prepare an intermediate of formula IVa, IVb or IVc which is substantially enantiomerically pure, and may be further converted into a corresponding product of formula Ia, Ib or Ic which is substantially enantiomerically and diastereomerically pure by a method described above using conditions which avoid racemization.

Inhibition Measurements:

The potency of compounds of the invention to act as elastase inhibitors was initially determined by the ability of a compound of the invention to inhibit the action of human leukocyte elastase (HLE) on a low molecular weight peptide substrate. The potency of an inhibitor is evaluated by obtaining a kinetic determination of the dissociation constant, $K_i$, of the complex formed from the intreraction of the inhibitor with HLE. The substrate used was the anilide methoxysuccinyl-alanyl-alanyl-prolyl-valine-p-nitroanilide as described by K. Nakajima et al. in the J. Biol. Chem., 254, 4027-4032 (1979) and by T. Teshima et al. in J. Biol. Chem., 257, No. 9, 5085-5091 (1982). The HLE enzyme used in these studies may be obtained from Elastin Products of St. Louis, Missouri or can be purified according to B. R. Viscarello et al. in Preparative Biochemistry, Vol. 13, pages 57-67, (1983) as follows, all work having been done in a cold room at 4°C.

Salt Extraction-DNase Treatment: The starting material, 193 g of purulent sputum, was homogenized with 200 ml of cold distilled water and centrifuged at 30,000 x gravity for 20 min. at 4°C. The supernatant was discarded and the pellet extracted with high salt and treated with DNase as per the method of D. Y. Twumasi et al. in J. Biol. Chem,., 252, 1917-1926 (1977). Chromatography on Elastin Agarose: The precipitate from the DNase digest was taken up in two 40 ml portions of 50 mM Tris, 1.0 M NaCl, pH 8; the suspension was centrifuged and the resulting supernatant was applied directly to a column of soluble elastin-Sepharose 4B (2.5 x 20 cm). The column was washed with equilibrating buffer (50 mM Tris, 50 mM NaCl, pH8.0) until the optical density at 280 nm ($OD_{280}$) of the eluate returned to baseline. Additional contaminating protein was eluted with two column volumes of 50 mM acetate, 1.0 M NaCl, pH 5.0. Elastase and cathepsin G (HLC-G) were finally eluted with 50 mM acetate, 1.0 M NaCl, 20% DMSO, pH 5.0. The column was developed at 6 ml/min with the collection of 10 ml fractions. The active fractions were pooled, dialyzed vs. two 6 liter changes of 50 mM acetate, 0.1 M NaCl, pH 5.5, and concentrated to 40 ml on an Amicon® ultrafiltration unit (YM-10 membrane). CM-Chromatography: The concentrated active fraction was applied to a column of CM-Sephadex® C-50 (2.2 x 10 cm) previously equilibrated with 50 mM acetate, 0.1 M NaCl, pH 5.5 and the column was then washed with this buffer to remove contaminating protein. Elution was continued with 50 mM acetate, 0.2 M NaCl, pH 5.5 and resulted in the displacement of a peak of activity assayed against Bz-Phe-Val-Arg-pNA. HLE was next eluted with the acetate buffer containing 0.45 M NaCl, while elution of HLC-G required the presence of 1.0 M NaCl in the buffer as described by R. Martodam et al. in Preparative Biochemistry, Vol. 9, pages 15-31 (1979). This

column was developed at 30 ml/hr with the collection of 5.5 ml fractions. From the thus purified HLE, a standard rate of production of p-nitroaniline was measured at 25°C spectrophotometrically in the visible spectrum at 410 nanometers with automatic data acquisition from a Cary 210 spectrophotometer obtained from Varian Associates. Reactions were initiated by injection of 10 microliters of the HLE solution into a 3 milliliter cuvette containing 2.89 milliliters of buffer (10 millimolar sodium phosphate, 500 millimolar NaCl, pH 7.6), 50 microliters substrate solution in DMSO, and 50 microliters of DMSO. Initial, steady-state reaction velocities of p-nitroaniline product were calculated by a fit of the experimental data to a linear dependence on time by linear least squares. This velocity, determined with no inhibitor present, was used as a standard in the calculation of inhibitor $K_i$ values.

In general, the peptide derivatives of the present invention were found to be "slow-binding" inhibitors of HLE and therefore required special methods of analysis to accurately determine $K_i$ values for their inhibition of HLE (see Williams, J. W. and Morrison, J. F., Meth. Enz. 63, 437 (1979) for a description of these methods.) In a typical experiment, 2.89 ml of buffer (10 millimolar sodium phosphate, 500 millimolar sodium chloride, pH 7.6), 50 microliters of inhibitor solution in DMSO, and 50 microliters of substrate solution in DMSO were added to a 3 milliliter cuvette. The cuvette was stoppered, inverted several times to mix its contents and maintained at (25°C) in the spectrophotometer. After a period of five minutes to allow the reaction solution to come to thermal equilibrium, 10 microliters of stock enzyme solution were added to the cuvette to initiate the reaction. Duplicate or triplicate runs were done at zero inhibitor concentration and at least three non-zero inhibitor concentrations. $K_i$ values were calculated according to methods outlined in the above reference by Williams and Morrison. The $K_i$values for selected compounds were less than $10^{-7}$M.

Animal Models

Animal models of emphysema include intratracheal (i.t.) administration of an elastolytic protease to cause a slowly progressive, destructive lesion of the lung. These lesions are normally evaluated a few weeks to a few months after the initial insult. However, these proteases also induce a lesion that is evident in the first few hours. The early lesion is first hemorrhagic, progresses to an inflammatory lesion by the end of the first 24 hours and resolves in the first week post insult. To take advantage of this early lesion, the following model may be used.

Hamsters are first lightly anesthetized with Brevital. Phosphate buffered saline (PBS) pH 7.4, either alone or containing 400 µg of human leukocyte elastase (HLE), is then administered directly into the trachea. Twenty-four hours later the animals are killed and the lungs removed and carefully trimmed of extraneous tissue. Following determination of wet lung weight, the lungs are lavaged with PBS and total lavagable red and white cells recovered are determined. The values for wet lung weights, total lavagable red cells and total lavagable white cells are elevated in a dose-dependent manner following administration of HLE. Compounds that are effective elastase inhibitors can prevent or diminish the severity of the enzyme-induced lesion resulting in lower wet lung weight and reduced values for total lavagable cells, both red and white, relative to administration of HLE alone. Compounds can be evaluated by administering them either with or at various times prior to administration of HLE to determine their utility in preventing an HLE lesion. Compounds of this invention produced statistically significant reductions in wet lung weight and total lavagable cells relative to HLE alone.

Compounds of the present invention exhibited activity in at least one of the tests described above under Inhibition Measurement or Animal Model. It should be noted that there was not always a direct correlation between the activities of the compounds measured as $K_i$ values in the Inhibition Measurement test and the reduced values for total lavagable cells and wet lung weight relative to the administration of HLE alone obtained in the Animal Model test. It is thought that the Animal Model test is more predictive of the activity of such compounds in the treatment of emphysema.

Pharmacokinetics: Male Syrian hamsters (80 to 120g) are injected intraveneously with the test compound. Prior to injection and at varying time periods thereafter, they are lightly anesthetized with ether and blood samples of approximately 0.2 ml each are withdrawn by cardiac puncture. The blood is expressed into 2 ml centrifuge tubes and allowed to clot for one hour. The sample is then centrifuged and the serum removed.

Drug levels are determined by first inactivating endogenous elastase inhibitors by incubation of 50 microliters of serum with an equal volume of buffer containing 5 mg/ml bovine pancreatic trypsin for 5 min. The trypsin inactivated serum (10 microliters) is then added to a 0.52 ml cuvette containing buffer made 20 nM with respect to HLE. After an additional 30 min. incubation, the reaction is started by the addition of substrate (350 microliters) (MeOSuc-Ala-Ala-Pro-Val-pNA, 1.6 mm) and the reaction monitored spectrophotometrically at a wavelength of 410 nM. For comparative purposes, serum persistance of the test compounds is determined in the following manner:

Percent inhibition of serum samples is calculated as follows:

$$\text{percent inhibition} = \frac{Vo - Vi}{Vo} \times 100$$

where Vo is the velocity obtained in the presence of control serum and Vi is the velocity of the inhibited reaction. Data are expressed as log percent inhibition vs. time post inhibitor administration. An approximate serum half-life ($t^1/_2$) is calculated from the resultant curve.

The compounds of the present invention may be administered to a warm-blooded animal in need thereof, particularly a human, for the treatment of conditions of pulmonary emphysema, atherosclerosis, rheumatoid arthritis, and osteo arthiritis, in particular for emphysema. The mode of administration may be oral, parenteral, including the subcutaneous deposit by means of an osmotic pump, or via a powdered or liquid aerosol. These may be conventionally formulated in an oral or parenteral dosage form by compounding about 10 to 250 mg per unit of dosage with conventional vehicle, excipient, binder, preservative, stabilizers, flavor or the like as called for by accepted pharmaceutical practice e.g. as described in U.S. Patent No. 3,755,340. For parenteral administration, a 1 to 10 ml intravenous, intramuscular or subcutaneous injection would be given containing about 0.02 to 10 mg/kg of body weight of a compound of the invention 3 or 4 times daily. The injection would contain a compound of the invention in an aqueous isotonic sterile solution or suspension or a solubilizing agent such as ethylenediaminetetraacetic acid (EDTA). In a powdered aerosol, compounds of the invention may be administered in the same manner as cromolyn sodium via a Spinhaler® turbo-inhaler device obtained from Fisons Corp. of Bedford, Massachusetts at a rate of about 0.1 to 50 mg per capsule, 1 to 8 capsules being administered daily for an average human. Each capsule to be used in the Spinhaler® contains the required amount of a compound of the invention with the remainder of the 20 mg capsule being a pharmaceutically-acceptable carrier such as lactose. In a liquid aerosol, the compounds of the invention are administered at the rate of about 100 to 1000 micrograms per "puff" or activated release of a standard volume of propellant. The liquid aerosol would be given at the rate of 1 to 8 puffs per day with variation in dosages due to the severity of the condition being treated, the weight of the patient and the particle size distribution of the aerosol since smaller particles will achieve greater lung penetration. Propellants, e.g., a fluorinated hydrocarbon or isobutane, containers, valves and actuators for liquid aerosols are described by L. Lachman et al, in "The Theory and Practice of Industrial Pharmacy", Lea and Febiger, Philadelphia (1976).

In the following Examples and throughout the specification, the following abbreviations and conventions are used: atm (atmospheres); bp (boiling point); °C (degrees Celsius) with all temperatures being in °C unless otherwise noted; g (grams) ; hr (hours); mg (milligrams); min (minutes); ml (milliliters); l (liters); mol (moles); mmol (millimoles); mp (melting point); bp (boiling point); N (normal); nm (nanometers); nM (nanomolar); satd or sat'd (saturated); aq (aqueous); conc (concentrated); x (times); room temperature (20-23°); DCC (1,3-dicyclohexylcarbodiimide); DMF (dimethylformamide); DMSO (dimethyl sulfoxide); $Et_2O$ (diethyl ether); EtOAc (ethyl acetate); HOAc (acetic acid); HOBT (1-hydroxybenzotriazole); MeOH (methyl alcohol); EtOH (ethyl alcohol); Pd/C (palladium on charcoal catalyst); pNA (paranitroanilide); THF (tetrahydrofuran); CBZ (benzyloxycarbonyl); t-BOC (tertiarybutyloxycarbonyl); DMF (dimethylformamide); TEA (triethylamine); DCC (1,3-dicyclohexylcarbodiimide); S.M. (starting material); NMM (N-methylmorpholine); ≦ (less than or equal to); TEA (triethylamine); TFA (trifluoroacetic acid); $Ac_2O$ (acetic anhydride); CDI (carbonyldiimidazole); WSCDI (water soluble carbodiimide:1-ethyl-3-(3-dimethylaminopropyl)carbodiimidehydrochloride); DMAP (4-dimethylaminopyridine); Dess-Martin periodinane (1,1,1-triacetoxy-2,1-benzoxiodol-3(3H)-one); HCl gas (gase ous HCl) - otherwise, HCl is an aqueous solution; Ø (phenyl group)l TLC (thin layer chromatography on silica gel unless otherwise specified); $R_f$ (relative mobility in TLC); HPLC (high pressure liquid chromatography), $t_R$ (HPLC retention time in min), FR (HPLC flow rate in ml/min); Col A (Zorbax® ODS analytical column, 4.6 mm x 25 cm); Col B (Phenomenex® Zorbax® C-8 analytical column, 4.6 mm x 35 cm); Col C (Altex Ultrasphere®/Octyl 10 mm I.D. x 25 cm 5 micron analytical and preparative column); flash chromatography (flash column chromatography on silica gel unless otherwise specified). Solvent ratios are given in volume:volume (v:v) terms. In addition, C, H, N, etc. (the conventional symbols for the elements) are used; 133.3 Pascals = 1 Torr as a conversion factor with 760 Torr = 14.7 pounds per square inch (psi); $^1H$ NMR (nuclear magnetic resonance) spectra were obtained using either a 80 MHz or 250 MHz instrument and tetramethylsilane (TMS) as an external standard (the solvent for the particular example is noted in the example), δ (parts per million downfield from TMS); with s (singlet); d (doublet); dd (doublet of doublets); m (multiplet).

Nomenclature: For uniformity and clarity, "amino acid sequence type" names are used whenever possible. In addition, amines of formula V, nitro compounds of formula VI and the N-substituents of C-terminal amides of formulae I-IV, VII and IX-X which are formally derived from V are numbered as shown in formulae V, VI and the partial formulae set out following them.

When needed or as noted, the preparations of various Examples were repeated if more material was required.

EXPLOSION WARNING: The nitro alcohols of formula VI and their nitroalkane precursors are potentially explosive. The compound of formula VI, $R^1=CH(CH_3)_2$ (Example 1b) is thermally unstable and, on a small scale, has been observed to decompose with considerable violence in the range 170-180°. Samples of the material have been safely distilled at reduced pressure (95-105°/2.0 torr). Recommendations for safe distillation include keeping the oil bath below 110°, never taking still residues below 15% of the original crude volume, and conducting the distillation behind a safety screen.

Example 1

2(RS),3(SR)-3-Amino-4-methyl-1,1,1-trifluoro-2-pentanol hydrochloride salt (Formula V, $R^1=CH(CH_3)_2$).

a. 2-Methyl-1-nitropropane.

A 5-liter, 3-necked, round-bottomed flask was equipped with a mechanical stirrer, thermometer, addition funnel and $N_2$ inlet. The flask was charged with $AgNO_2$ (1006.8 g) in $Et_2O$ (2.5 liter), and isobutyl iodide (927.2 g) was placed in the addition funnel. Both the flask and the addition funnel were wrapped in aluminum foil to protect the reaction from light. After the stirred suspension was cooled to approximately 5° (ice bath), dropwise addition of the iodide over a 2 hr period was begun. The reaction temperature was maintained at or less than 5° throughout the course of the addition. When the addition was complete, the reaction vessel was packed in ice and allowed to warm slowly to room temperature overnight. NMR analysis of an aliquot taken from the reaction mixture after 48 hr of stirring demonstrated that all of the isobutyl iodide had been consumed. The reaction mixture was filtered through diatomaceous earth to remove silver salts and the filter cake was washed with $Et_2O$ (3 x 500 ml). The combined filtrates were dried ($MgSO_4$), filtered and concentrated on a rotary evaporator (bath temp = 35°) to about 600 ml. Fractional distillation (atmospheric pressure) (caution: potentially explosive) gave the purified nitro compound (350.4 g, 68% yield); bp 135°-142°.

b. 2(RS),3(SR)-4-Methyl-3-nitro-1,1,1-trifluoro-2-pentanol (Formula VI, $R^1 = CH(CH_3)_2$).

See EXPLOSION WARNING before Example 1. A 3-liter, 3-necked, round-bottomed flask equipped with a mechanical stirrer and $N_2$ inlet was charged with $K_2CO_3$ (470.0 g), the product of Example 1a (350.0 g) and finally trifluoroacetaldehyde ethyl hemiacetal (708.0 g). The mixture was vigorously stirred at room temperature for 76 hr, at which time $^1H$-NMR demonstrated the nearly complete consumption of the nitroalkane. The reaction mixture was diluted with $CH_2Cl_2$ and filtered. The filtrate was treated with aqueous HCl until pH = 3. The layers were separated and the aqueous layer was washed with $CH_2Cl_2$ (500 ml). The combined $CH_2Cl_2$ portions were washed with $H_2O$ (1 l) and brine (1 l). Drying ($MgSO_4$) and concentration gave 854.6 g of crude product as a yellow oil. $^1H$-NMR showed the two diastereomeric nitro alcohols (present in the ratio of about 3:1 as quantified by integration of the alcohol protons which consistently appear in the range δ 6.0-6.5 when run in acetone-$d_6$) contaminated by solvents and small amounts of starting materials.

Distillation at reduced pressure gave the following fractions:

| | Wt. | BP (°C) | |
|---|---|---|---|
| A | 191.7 g | 42°-50°/atm. | S.M. + solvents |
| B | 34.8 g | 35°/1 torr - 45°/.5 torr | |
| C | 213.6 g | 45°/.5 torr - 95°/1.5 torr | |
| D | 337.8 g | 95°/1.5 torr - 105°/2 torr | |
| E | 114.0 g | trap volatiles | |

To obtain the major diastereomeric pair (which crystallizes from the mixture of diastereomers, as well as from cold pentane, to yield colorless needles) in a substantially pure state and to advance only this material, fraction C from the above distillation was allowed to crystallize overnight in a refrigerator. The product was collected, washed with cold pentane and dried for several hours in a vacuum oven (Caution|This material is somewhat volatile and significant quantities can be lost under extended vacuum treatment) to give 52.0 g of substantially pure material. The fractions known (by NMR) to contain significant quantities of the desired isomer were repetitively treated in this fashion (and redistilled to provide new fractions further enriched in the desired diastereomer) to eventually obtain a total of 197.7 g of substantially pure nitro alcohol. This amount represents the type of work done, but it does not reflect the upper limit of the yield.

c. 2(RS),3(SR(-3-Amino-4-methyl-1,1,1-trifluoro-2-pentanol hydrochloride salt.

Anhydrous EtOH (232 ml) was added to 10% Pd/C catalyst (2.30 g) under $N_2$. (Less active catalysts (e.g., 10% Pd/$BaSO_4$, wet 10% Pd/C) or insufficient reaction times may lead to the production of one or more by-products.) The product of Example 1b (22.93 g, 0.144 mol) was added and the resultant mixture was placed on a Parr hydrogenation apparatus (3.7 bar, 55 psig $H_2$) overnight. Catalyst was removed by filtration through diatomaceous earth. The filter cake was then washed with EtOH. HCl gas was bubbled through the combined filtrates until approximately 8 g (about 0.22 mol) was absorbed. The solution was concentrated on a rotary evaporator and the resultant residue was concentrated several times from $Et_2O$ to obtain a white solid. The solid was washed with $Et_2O$ and dried overnight in a vacuum oven to yield 20.79 g (88%) of the title amine hydrochloride. For the mp, with slow heating the material softened at 90° and melted at 118°-120°. When a sample was plunged into a bath preheatd to 110°, it melted instantaneously.

Example 2

2(RS),3(SR)-L-valyl-N-[3-(2-hydroxy-4-methyl-1,1,1-trifluoropentyl)]-L-polinamide (Formula IVb, $R^1 = CH(CH_3)_2$, $R^2 = CH(CH_3)_2$, $R^4 = H$).

a. N-[(Benzyloxy)carbonyl]-L-valyl-L-proline methyl ester.

A solution of CBZ-L-valine (100.0 g) in DMF (1 l, dried over sieves) was added to a 3-liter, 3-necked, round-bottomed flask equipped with a mechanical stirrer, $N_2$ inlet and a thermometer. The reaction was cooled to 0° and HOBT hydrate (108.1 g) was added. Approximately 15 min of stirring were allowed before adding a DMF (500 ml) slurry of L-proline methylester hydrochloride (66.2 g) and TEA (41.8 g) in one portion. Additional DMF (500 ml) was used to complete the transfer of the slurry. DCC (90.8 g) was added to the reaction and was washed down with DMF (100 ml). The reaction was stirred for 3 hr at 0° before allowing it to warm to room temperature and stir for 3 days. The reaction mixture was then filtered and the filtrate concentraed at reduced pressure. The filter cake was washed with EtOAc (3 x 1 l), and concentration of the resultant filtrate gave material that was combined with the residue from concentration of the DMF solution. The combined product mixture (about 2.5 l) was diluted with $Et_2O$ (2 l) and stored in the refrigerator overnight. Precipitate was removed by filtration. When the filtrate was washed with 1 N HCl (1 l), additional precipitate formed and was removed by filtration. The filtrate was then washed with 1 N HCl (1 l), $H_2O$ (0.5 l), saturated $NaHCO_3$ (2 x 1 l) and brine (0.5 l). Drying ($MgSO_4$) and concentration gave 587.2 g of crude product. This material was flash chromatographed on silica gel (3.5 kg) with gradient elution ($CH_2Cl_2$ to MeOH:$CH_2Cl_2$ (5:95)). Mixed fractions were subjected to repeated chromatography to remove impurities. Combination of the fractions containing the desired product gave 500.7 g (87%) of material contaminated with a small amount of low $R_f$impurity; TLC, $R_f = 0.37$, silica gel, $Et_2O$:hexane (3:1); $R_f = 0.53$, silica gel, MeOH:$CHCl_3$ (5:95).

b. N-[(Benzyloxy)carbonyl]-L-valyl-L-proline.

A methanolic (4 l) solution of the product of Example 2a (500.7 g) was added to a 12-liter, 3-necked, round-bottomed flask equipped with a mechanical stirrer and an $N_2$ inlet. To the stirred solution was added 1 N NaOH (1.4 l), bringing the pH to approximately 13. After the reaction had stirred for 3 hr, the pH dropped to 11. Additional 1N NaOH (0.1 l) was used to bring the pH to 12, and the reaction was stirred overnight at room temperature. MeOH was removed from the reaction mixture by concentration on a rotary evaporator. During the course of the solvent removal, a total of 1 liter of $H_2O$ was added to reduce the concentration of the base. The aqueous solution was washed with $Et_2O$ before acidifying with 1 N HCl (1.5 l) to pH of approxiately 3.5. The layers were separated and the aqueous layer was extracted with EtOAc (3 x 1 l). The combined organics were washed with brine (1 l), dried ($MgSO_4$) and concentrated to give 493.0 g (100%) of product as a white solid; TLC, $R_f = 0.51$, silica gel, MeOH:$CHCl_3$ (5:95) with added AcOH.

c. 2(RS),3(SR) = [(Benzyloxy)carbonyl]-L-valyl-N-[3-(2-hydroxy-4-methyl-1,1,1-trifluoropentyl)]-L-prolinamide.

The product of example 2b (105.3 g) was dissolved in dry THF (1.5 liters) under $N_2$ in a 3-liter, 3-necked, round-bottomed flask equipped with a mechanical stirrer, thermometer, $N_2$ inlet and an addition funnel. The solution was cooled to -35° and treated with 1 equivalent (34 ml) of NMM. Isobutyl chloroformate (39 ml) was added dropwise over 20 min while maintaining the temperature $\leqq$-35°. After the addition was complete, the reaction was stirred for 1 hr at -35°. A second equivalent of NMM (34 ml) was added. The product of Example 1c (62.8 g) in THF (300 ml) was then added at such a rate that the temperature was maintained at $\leqq$-35°. After the addition was complete, the temperature was kept $\leqq$-35° for 1 hr before the mixture was allowed to warm to room temperature with stirring overnight. The reaction mixture was filtered and the filter cake was washed with THF (1 l). The combined filtrates were concentrated to give 189 g of crude product. This material was flash chromatographed on silica gel (5 1) and eluted with THF:toluene (1:9). Once product began to elute, the solvent polarity was altered in gradient fashion: THF:toluene (15:85); THF:tolune (20:80); and, finally MeOH:THF:toluene (2.5:30:70) MeOH use was minimized to prevent the elution of low $R_f$ impurities). Concentration of the column fractions followed by drying under vacuum overnight gave 12.0 g (8%) of slightly impure product and 131.6 g (87%) of substantially pure material. (NOTE: Solutions of this material when taken to complete dryness yielded a foam that eventually solidified under extended vacuum treatment. Care had been taken to accomplish this operation in a large enough flask to accomodate the expansion of the foam.) TLC, $R_f = 0.25$, silica gel, MeOH:$CHCl_3$ (5:95); $R_f = 0.37$, silica gel, THF:tolune (20:80). When the material is spotted lightly, the two isomers resolved to give spots at $R_f = 0.37$ and $R_f = 0.46$, silica gel, THF:toluene (20:80).

(NOTE: Maintenance of the internal temperatures quoted in this procedure appears to be crucial for obtaining substantially pure product.)

d. 2(RS),3(SR)-L-Valyl-N-[3-(2-hydroxy-4-methyl-1,1,1-trifluoropentyl)]-L-prolinamide.

The product of Example 2c (131.6 g) was dissolved in EtOH (750 ml) and combined with 10% Pd/C catalyst (50% $H_2O$, 13.0 g) under $N_2$ in a 2-liter Parr hydrogenation bottle. The reaction mixture was shaken under a 3.7 bar, 55 psig atmosphere of $H_2$ on a Parr apparatus. Repressurizing with $H_2$ was continued until no further uptake was observed. Examination of the reaction by TLC showed complete consumption of starting material. The reaction mixture was filtered through diatomaceous earth and concentrated to a foam. This material was triturated with $Et_2O$, filtered, and dried to give 81.4 g (84%) of the title compound as a light grey solid; TLC,

$R_f$=0.41, silica gel, $CHCl_3$:MeOH (10:1).

Example 3

3(RS)-[4-[(N'-Phenylureido)sulfonyl]benzoyl]-L-valyl-N-[3-(1,1,1-trifluoro-4-methyl-2-oxopentyl)]-L-prolinamide (Formula Ib, $R^1$=$CH(CH_3)_2$, $R^2$=$CH(CH_3)_2$, $R^4$=H, $R^7$=Ø, A=$R^7$.NH.CO.NH.S($O_2$)- para to -CO-).

a. 2(RS),3(SR)-[4-(Aminosulfonyl)benzoyl]-L-valyl-N-[3-(2-hydroxy-4-methyl-1,1,1-trifluoropentyl)]-L-prolinamide. Formula IXb, $R^1$=$CH(CH_3)_2$, $R^2$=$CH(CH_3)_2$, $R^4$=H, M=$H_2$NS($O_2$)- para to -CO-).

To a reaction vessel containing 4-carboxybenzene sulfonamide (1.24 g), the product of Example 2 (1.49 g), MAP (0.60 g) and DMF:$CH_2Cl_2$ (1:1, 40 ml) was added WSCDI (0.94 g). The reaction was stirred overnight under $N_2$ and at room temperature before it was washed with 1N HCl (2 x 50 ml) and brine (1 x 50 ml), dried ($Na_2SO_4$), and concentrated under vacuum to give the crude product (3.11 g). The crude product was purified by flash chromatography on acidic silica gel (MeOH:$CH_2Cl_2$ (15:85)) to afford the pure product (0.52 g, 24.8%); TLC, $R_f$=0.49-0.65, $CH_2Cl_2$:MeOH:HOAc (89.5:10:0.5).

b. 3(RS)-[4-(Aminosulfonyl)benzoyl]-L-valyl-N-[3-(1,1,1-trifluoro-4-methyl-2-oxopentyl)]-L-prolinamide (Formula Xb, $R^1$=$CH(CH_3)_2$, $R^2$=$CH(CH_3)_2$, $R^4$=H, M=$H_2N(SO_2)$- para to -CO-).

To a reaction vessel containing the product of Example 3a (250 mg), Dess-Martin periodinande (324 mg) and $CH_2Cl_2$ (10 ml) was added TFA (86.7 mg, 58.5 μl). After the reaction was stirred under $N_2$ and at room temperature for 3 hours, it was partitioned between EtOAc (50 ml) and satd $NaHCO_3$ (50 ml) containing $Na_2S_2O_3$ (841 mg). After the organic phase was separated, the aqueous layer was extrated with EtOAc (2 x 50 ml). The combined organic phase was washed with brine (2 x 50 ml), dried ($Na_2SO_4$), and concentrated under vacuum to provide a crude product (230 mg). The crude product was purified by flash chromatography (acidic silica gel; MeOH:$CH_2Cl_2$ (10: 90)) to give pure product (180 mg, 72.3%); TLC, $R_f$=0.60-0.69, MeOH:$CH_2Cl_2$ (10:90).

analysis calculated for:
$C_{23}H_{31}F_3N_4O_6S$.0.5 $H_2O$: C, 49.54; H, 5.78; N, 10.05
Found: C, 49.53; H, 5.77; N, 9.51

c. 3(RS)-[4-[(N'-Phenylureido)sulfonyl]benzoyl]-L-valyl-N-[3-(1,1,1-trifluoro-4-methyl-2-oxopentyl)]-L-prolinamide (Formula Ib, $R^1$=$CH(CH_3)_2$, $R^2$=$CH(CH_3)_2$, $R^4$=H, $R^7$=Ø, A=$R^7$.NH.CO.NH.S($O_2$)-para to -CO-).

Phenyl isocyanate (21 mg, 0.168 mmol) was added to a stirred solution of the product of Example 3b (88 mg, 0.16 mmol), water (2.0 ml), acetone (0.66 ml) and 1N NaOH (0.32 ml, 0.32 mmol) at about 10°. The reaction mixture was stirred at about 10° for 2.5 hr, then allowed to warm to room temperature. Water (20 ml) was added, and the mixture was filtered. The filtrate was washed with EtOCc and made acidic (pH=2) with 1N HCl. This acidic mixture was extracted with EtOAc (2 x 20 ml). The EtOAc extracts were washed with brine, dried ($MgSO_4$), concentrated and dried under vacuum to give the title product (55.8 mg, 52%) as a white powder; HPLC, $t_R$=12.04 and 17.26, Col B, $H_2O$:$CH_3CN$:THF:TFA (55:35:15:0.1), FR=1.0.

Analysis calculated for:
$C_{30}H_{36}F_3N_5O_7S$.0.5 $H_2O$: C, 53.25; H. 5.51; N, 10.35
Found: C, 53.43; H, 5.35; N, 9.99

Example 4

3(RS)-[4-[[N'-(4-Chlorophenyl)ureido]sulfonyl]benzoyl]-L-valyl-N-[3-(1,1,1-trifluoro-4-methyl-2-oxopentyl)]-L-prolinamide (Formula Ib, $R^1$=$CH(CH_3)_2$, $R^2$=$CH(CH_3)_2$, $R^4$=H, A=$R^7$=4-ClØ, $R^7$.NH.CO.NH.S($O_2$)- para to -CO-).

4-Chlorophenyl isocyanate (266 mg) was added in five portions over 5 hr and 1N NaOH (1.73 ml) was added concurrently to a solution of the product of Example 3b (190 mg), water (4 ml) and acetone (2 ml), at about 10°. The mixture was then stirred at room temperature for 30 min and filtered. The filtrate was diluted with water (40 ml), washed with EtOAc (75 ml) and acidified to pH 2 with 1N HCl. The resulting milky solution was extracted with EtOAc (100 ml). The ethyl acetate phase was washed with brine, dried ($Na_2SO_4$), and concentrated under vacuum to give the crude product (149 mg) which was purified by flash chromatography on acidic silica gel (MeOH:$CH_2Cl_2$ (4:96)) to give the product (71 mg, 30%) as a white foam; TLC, $R_f$= 0.45-0.55, $CH_2Cl_2$:MeOH:HOAc (95:5:0.1).

Analysis calculated for
$C_{30}H_{35}F_3ClN_5O_7S.H_2O$: C, 50.03; H, 5.18; N, 9.72
Found: C, 49.80; H, 4.80; N, 9.70

3(RS)-[4-[[N'-(1-Naphthalenyl)ureido]sulfonyl]benzoyl]-L-valyl-N-[3-(1,1,1-trifluoro-4-methyl-2-oxopentyl)]-L-prolinamide (Formula Ib, $R^1$=$CH(CH_3)_2$, $R^2$=$CH(CH_3)_2$, $R^4$=H, $R^7$-1-naphthalenyl, A=$R^7$=NH.CO.NH.S($O_2$)-para to -CO-).

To a stirred solution of the product of Example 3b (175 mg) in $H_2O$ (4 ml) and acetone (2 ml) was added 1N NaOH (0.7 ml) at 10°. The mixture was then treated with 1-naphthyl isocyanate (0.06 g). At 30 minute intervals

five additional portions of 1-naphthyl isocyanate (0.06 g each) were added along with sufficient mixture was warmed to room temperature and stirred for an additional 30 minutes and filtered. The filtrate was washed with EtOAc (50 ml) acidified with 1N HCl to pH 4, and extracted wtih EtOAc (75 ml). The EtOAc extract was washed (brine), dried ($Na_2SO_4$), and concentrated under vacuum to give crude product (80 mg) which was purified by flash chromatography twice on acidic silica gel (MeOH:$CH_2Cl_2$ (3:97)) to give the product (36.5 mg, 16%); TLC, $R_f$=0.5-0.58, MeOH:$CH_2Cl_2$:HOAc (5:95:0.1).
Analysis calculated for
$C_{34}H_{38}F_3N_5O_7S.2H_2O$: C, 54.18; H, 5.62; N, 9.29
Found: C, 54.47; H, 5.48; N, 9.54

Example 6

3(RS)-[4-[N'-(Phenylsulfonyl)ureido]benzoyl]-L-valyl-N-[3-(1,1,1-trifluoro-4-methyl-2-oxopentyl)]-L-prolinamide (Formula Ib, $R^1$=CH($CH_3$)$_2$, $R^2$=CH($CH_3$)$_2$, $R^4$=$R^8$=H, $R^9$=Ø, A=$R^9$.S($O_2$).NH.CO.N$R^8$- para to -CO-).

a. 4-[N'-(Phenylsulfonyl)ureido]benzoic acid.
Benzenesulfonyl isocyanate (2.00 g, 1.46 ml) was added to a reaction vessel containing p-aminobenzoic acid (1.50 g), dry THF (75 ml) and NMM (1.10g, 1.20 ml). After the reaction mixture was stirred overnight under $N_2$ and at room temperature, it was diluted with $CH_2Cl_2$ (125 ml), acidified to pH 4.0 with 1N HCl, and the phases were separated. The remaining organic phase was washed with 1N HCl (2 x 100 ml) and brine (1 x 100 ml), dried ($Na_2SO_4$) and concentrated in vacuo to give the crude product (3.50 g) which was purified by ether trituration (200 ml) to give the pure product as a white powder (2.79 g, 79.7%); TLC, Rf=0.12, $CH_2Cl_2$:MeOH:HOAc (96.5:3:0.5).

b. 2(RS),3(SR)-[4-[N'-(Phenylsulfonyl)ureido]benzoyl]-L-valyl-N-[3-(1,1,1-trifluoro-2-hydroxy-4-methylpentyl)]-L-prolinamide (Formula VIIb, $R^1$=CH($CH_3$)$_2$. $R^2$=CH($CH_3$)$_2$, $R^4$=$R^8$=H, $R^9$=Ø A=$R^9$.S($O_2$)NH.CO.N$R^8$- para to -CO-).
To a reaction vessel containing the product of Example 6a (0.53g), the product of Example 2d (0.63g), DMAP (0.24g) and dry $CH_2Cl_2$:DMF(1:1, 20 ml) was added WSCDI (0.38 g). The reaction mixture was stirred overnight under $N_2$ and at room temperature before it was diluted with $CH_2Cl_2$ (50 ml), washed with 1N HCl (2 x 50 ml) and brine (1 x 50 ml), dried ($Na_2SO_4$) and concentrated under pump vacuum to give the crude product (0.97 g). The crude product was purified by flash chromatography on acidic silica gel eluting with MeOH:$CH_2Cl_2$ (5:95) to give 0.43 g (38.74%) of the product; TLC, Rf=0.29-0.40, $CH_2Cl_2$: MeOH:HOAc (94.5:5:0.5).

c. 3(RS)-[4-[N'-(Phenylsulfonyl)ureido]benzyl]-L-valyl-N-[3-(1,1,1-trifluoro-4-methyl-2-oxo-pentyl)]-L-prolinamide (Formula Ib, $R^1$=CH($CH_3$)$_2$, $R^2$=CH($CH_3$)$_2$, $R^4$=$R^8$=H, $R^9$=Ø, A=$R^9$.S($O_2$).NH.CO.N$R^8$-para to -CO-).
To a reaction vessel containing the product of Example 6b (200 mg), Dess-Martin periodinane (253 mg) and $CH_2Cl_2$ (10 ml) was added TFA (68 mg, 46 µl). The reaction mixture was stirred under $N_2$ and at room temperature overnight before it was partitioned between EtOAc (50 ml) and satd $NaHCO_3$ (50 ml) and $Na_2S_2O_3$ (0.66 g). After the organic layer was separated, the aqueous layer was acidified to pH 2.0 with 6N HCl and extracted with $CH_2Cl_2$. The $CH_2Cl_2$ phase was washed with 2% $NaHCO_3$ (30 ml) and brine (20 ml), dried ($Na_2SO_4$) and concentrated in vacuo to give the crude product (72 mg). The crude product was purified by flash chromatography on acidic silica gel eluting with MeOH:$CH_2Cl_2$ (5:95) to give 50 mg (25%) of the title product; TLC, Rf=0.42-0.49, $CH_2Cl_2$:MeOH:HOAc (94.5:5:0.5).
Analysis calculated for
$C_{30}H_{36}F_3N_5O_7S.1.5\ H_2O$: C, 51.86; H, 5.66; N, 10.08
Found: C, 51.98; H, 5.38; N, 9.73

Example 7

3(RS)-[4-[N'-[(4-Chlorophenyl)sulfonyl]ureido]benzoyl]-L-valyl-N-[3-(1,1,1-trifluoro-4-methyl-2-oxopentyl)]-L-prolinamide (Formula Ib, $R^1$=CH($CH_3$)$_2$, $R^2$=CH($CH_3$)$_2$, $R^4$=$R^8$=H, $R^9$=4-ClØ, A=$R^9$.S($O_2$).NH.CO.N$R^8$- para to -CO-).

a. 4-[N'-[(4-Chlorophenyl)sulfonyl]ureido]benzoic acid.
4-Chlorobenzenesulfonyl isocyanate (2.38 g) was added to a reaction vessel containing 4-aminobenzoic acid (1.50 g), dry THF (50 ml) and NMM (1.10) g). After the reaction mixture was stirred overnight under $N_2$ at room temperature, it was diluted with $CH_2Cl_2$ (100 ml), acidified to pH 2.0 with 1N HCl, and the phases separated. The organic phase was washed with 1N HCl (2x50 ml) and brine (1x100 ml), dried ($Na_2SO_4$) and concentrated under vacuum to give the crude product (4.05 g) which was purified by ether trituration to give the pure product as a white powder (3.07 g, 79.3%); TLC, $R_f$=0.39, $CH_2Cl_2$:MeOH:HOAc (95:5:0.1).

b. 2(RS),3(RS)-[4-[N'-[(4-Chlorophenyl)sulfonyl]ureido]benzoyl]-L-valyl-N-[3-(1,1,1-trifluoro-2-hydroxy-4-methylpentyl)]-L-prolinamide (Formula VIIb, $R^1=CH(CH_3)_2$, $R^3=CH(CH_3)_2$, $R^4=R^8=H$, $R^9=4$-Cl, $A=R^9.S(O_2).NH.CO.NR^8$- para to -CO-).

To a solution of the product of Example 7a (1.00 g), the product of Example 2d (1.09 g), and DMAP (0.38 g) in dry $CH_2Cl_2$:DMF (1:1, 40 ml) was added WSCDI (0.59 g). The reaction mixture was stirred overnight under $N_2$ at room temperature before it was washed with 1N HCl (75 ml), and brine (50 ml), dried ($Na_2SO_4$) and concentrated under pump vacuum to give the crude product (2.35 g). The crude product was purified by flash chromatography an acidic silica gel eluting with MeOH:$CH_2Cl_2$ (5:95) to give 1.87 g (94%) of the product; TLC, $R_f=.035$-0.48, $CH_2Cl_2$:MeOH: HOAc (95:5:0.1).

c. 3(RS)-[4-[N'-[(4-Chlorophenyl)sulfonyl]ureido]benzoyl]-L-valyl-N-[3-(1,1,1-trifluoro-4-methyl-2-oxopentyl)]-L-prolinamide (Formula Ib, $R^1=CH(CH_3)_2$, $R^2=CH(CH_3)_2$, $R^4=R^8=H$, $R^7=4$-Cl, $A=R^7.S(O_2).NH.CO.NR^8$- para to -CO-).

To a reaction vessel containing the product of Example 7b (810 mg), Dess-Martin periodinane (975 mg) and $CH_2Cl_2$ (50 ml) was added TFA (259 mg, 175 µl). The reaction mixture was stirred under $N_2$ at room temperature for 2 hr. The mixture was concentrated under vacuum to yield the crude product (1.7 g) as a tan solid. The crude product was purified by flash chromatography (twice) on acidic silica gel eluting with MeOH:$CH_2Cl_2$ (5:95) to give 520 mg of the title product; TLC, $R_f=0.4$-0.5, $CH_2Cl_2$:MeOH:HOAc (95:5:0.1).
Analysis calcualted for
$C_{30}H_{35}F_3N_5O_7S.1.0\ H_2O$: C, 50.03; H, 5.18; N, 9.72
Found: C, 49.85; H, 5.06; N, 9.54

## Example 8

3(RS)-(4-Carboxybenzoyl)-L-valyl-N-[3-(1,1,1-trifluoro-4-methyl-2-oxopentyl)]-L-prolinamide (Formula Xb, $R^1=CH(CH_3)_2$, $R^2=CH(CH_3)_2$, M=COOH para to -CO-).

a. 4-Methoxycarbonylbenzenecarboxylic acid.

Concentrated sulfuric acid (277.5 ml, 5.2 mol) was added dropwise over 1/2 hr to a stirred solution of chromium (VI) oxide (299.25 g, 2.99 mol) and water (925 ml) at 0°. The resulting solution of added dropwise over 1 hr to a stirred solution was methyl-4-(hydroxymethyl)benzoate (92.5 g, 0.564 mol) and acetone (4.6 l) at 0°. The reaction mixture was allowed to warm to room temperature and stirred overnight. The supernatant was decanted before the black tar-like residue was extracted with acetone. The decanted supernatant and acetone extracts were combined and concentrated under vacuum to leave a dark brown residue which was triturated with cold water (4 l). The precipitate which formed was collected, washed three times with water (1 l), and dried to give 94.6 g (94%) of the title compound as white crystals, m.p. 218-221°C.

b. 2(RS),3(SR)-4-(Methoxycarbonyl)benzoyl-L-valyl-N-[3-(1,1,1-trifluoro-2-hydroxy-4-methyl-pentyl)]-L-prolinamide (Formula IXb, $R^1=CH(CH_3)_2$, $R^2=CH(CH_3)_2$, $R^4=H$, $M=COOR^a$, $R^a=CH_3$, M para to -CO-).

WSCDI (10.27 g, 53.6 mmol) was added to a stirred solution of a compound prepared according to Example 2d (17.54 g, 47.8 mmol), the product of Example 8a (8.6 g, 47.8 mmol), HOBT (12.86 g, 95.3 mmol) and dry THF (400 ml) at 0° under nitrogen. The resulting reaction mixture was stirred at 0° for 1 hr; then it was allowed to come to room temperature and to stir overnight. The THF was removed under vacuum to leave an oily residue which was dissolved in EtOAc. The EtOAc solution was washed (1N HCl, satd aq $NaHCO_3$, and brine), dried ($MgSO_4$), filtered, and concentrated under vacuum to give 24.45 g of the crude product as a dry white foam. Purification by flash chromatography ($CHCl_3$:MeOH (97:3)) produced the title compound (79%); HPLC, $t_R=4.62$ & 5.80, Col A, $H_2O$:$CH_3CN$ (55:45); Fr=3.0.

c. 3(RS)-[4-(Methoxycarbonyl)benzoyl]-L-valyl-N-[3-(1,1,1-trifluoro-4-methyl-2-oxopentyl)]-L-prolinamide (Formula Xb, $R^1=CH(CH_3)_2$, $R^2=CH(CH_3)_2$, $R^4=H$, $M=COOR^a$, $R^a=CH_3$, M para to -CO-).

A preferred method employing the oxidation described in D. B. Dess and J. C. Martin, J. Org. Chem., 48, 4155-4156(1983) for the preparation of the title compound is as follows: To a stirred solution of Dess-Martin periodinane (6.76 g, 15.96 mmol) and a product prepared using the method of Example 8b (6.49 g, 12.25 mmol) in dry $CH_2Cl_2$ (80 ml) under nitrogen was added TFA (1.82 g, 1.23 ml, 15.96 mmol). After the reaction mixture had been stirred overnight at room temperature, $Et_2O$ (about 3 to 5 times the volume of $CH_2Cl_2$) was added, and the mixture was poured into an aq solution of satd $NaHCO_3$ and $Na_2S_2O_3$ (17.63 g, 111.51 mmol). After 15 min of stirring, the organic layer was separated, washed (satd $NaHCO_3$, brine), dried ($Na_2SO_4$), filtered, and concentrated to give the title product as a white foam (6.42 g, 99.4%); TLC, $R_f=$ 0.67 & 0.76; MeOH:$CHCl_3$ (3:97); HPLC, $t_R=4.64$ & 6.84, Col A, $H_2O$:$CH_3CN$ (60:40), FR=2.0.

d. 3(RS)-(4-Carboxybenzoyl)-L-valyl-N-[3-(1,1,1-trifluoro-4-methyl-2-oxopentyl)]-L-prolinamide (Formula Xb, $R^1$-$CH(CH_3)_2$, $R^2=CH(CH_3)_2$, $R^4=H$, M=COOH para to -CO-).

1N NaOH (26 ml) was added to a stirred solution of the ester prepared using a procedure similar to that of Example 8c (7.0 g, 13.6 mmol) in MeOH (100 ml). Water (70 ml) was added, and the solution was stirred at room temperature for 3 hr. The MeOH was removed under vacuum, and the remaining aqueous solution was

extracted with EtOAc. After the layers were separated, the aqueous layer was acidified with 1N HCl. The resulting suspension was extracted with EtOAc; the combined extracts were washed with brine, dried ($MgSO_4$), and concentrated to give the title compound as a white solid (6.01 g); HPLC, $t_R$=3.50 and 4.26, Col A, $CH_3CN$:0.01M $K_2HPO_4/H_3PO_4$ [pH3] (55:45).
Analysis calculated for:
$C_{24}H_{30}F_3N_3O_6.H_2O$: C, 54.23; H, 6.07; N, 7.91
Found: C, 53.93; H, 5.93; N, 7.66

Example 9

3(RS)-[4-[(2-Pyridylsulfonylamino)carbonyl]benzoyl-L-valyl-N-[3-(1,1,1-trifluoro-4-methyl-2-oxopentyl)]-L-prolinamide (Formula Ib, $R^1$=$CH(CH_3)_2$, $R^2$=$CH(CH_3)_2$, $R^4$=H, Q=2-pyrdiyl, B=-$S(O_2)$.NH.CO-, A=Q.B- para to -CO-).

a. 2-Pyridinesulfonyl chloride.
Water (13.5 ml) and conc HCl(4.1 ml) were added to a cooled (5° C) solution of 2-mercaptopyridine (0.67g, 6.0 mmol) in $CH_2Cl_2$ (30 ml). The mixture was vigorously stirred and 5% aq NaOCl (69 ml) was added over 15 min. The phases were separated, the aqueous phase was extracted with $CH_2Cl_2$, the combined organic phases were washed with aqueous $NaHSO_3$, dried ($MgSO_4$) and concentrated to give 400 mg of a pale yellow semi-solid which was used without purification.

b. 2-Pyridinesulfonamide.
The product of Example 9a was dissolved in a mixture of $CH_2Cl_2/Et_2O$(1:2) and cooled to -30° C. Gaseous $NH_3$ was bubbled into the solution for 10 min. The reaction was stirred overnight and allowed to warm to room temperature. The reaction mixture was filtered; and the precipitate was suspended in EtOAc/EtOH (3:1) and filtered. The combined filtrates were concentrated to give the product as a white solid (308 mg); TLC, $R_f$=0.44, MeOH:$CHCl_3$ (5:95).

c. 3(RS)-[4(2-Pyridylsulfonylamino)carbonyl]benzoyl-L-valyl-N-[3-(1,1,1-trifluoro-4-methyl-2-oxopentyl)]-L-prolinamide (Formula Ib, $R^1$=$CH(CH_3)_2$, $R^2$=$CH(CH_3)_2$, $R^4$=H, Q=2-pyridyl, B=-$S(O_2)$.NH.CO-, A=Q.B- para to -CO-).
WSCDI (0.25g, 1.3 mmol) was added to a mixture of the product of Example 8d (0.5g, 1.08 mmol), the product of Example 9b (0.17g, 1.08 mmol) and DMAP (0.13g, 1.3 mmol) in $CH_2Cl_2$ (10 ml) and stirred at room temperature for 3 days. The reaction mixture was concentrated; and the residue was taken up in EtOAc and washed with 1N HCl and brine, dried ($Na_2SO_4$) and concentrated. The product was flash chromatographed on acidic silica gel eluted with a gradient of $CHCl_3$, MeOH:$CHCl_3$ (1:99), MeOH:$CHCl_3$ (2:98), MeOH:$CHCl_3$ (5:95) to give the title product as a white solid (0.33g, 51%); HPLC, $t_R$=3.14 and 4.63, Col A, $CH_3CN$: 0.01M $K_2HPO_4/H_3PO_4$ [pH 3] (35:65), FR=2.0; NMR (DMSO-$d_6$): 8.71 (2H, m), 8.62 (0.6H, m), 8.14 (2H, d, J=3.88), 7.95 (4H, s), 7.68 (1H, m), 7.48 (0.4H, m), 4.66 (0.3H, m), 4.45 (2.3H, m), 4.02 (0.4H, m), 3.88 (1H, m), 3.64 (1H, m), 1.99 (6H, m), 0.92 (12H, m).
Analysis calculated for:
$C_{29}H_{34}F_3N_5O_7S.1.5\ H_2O$: C, 51.17; H, 5.48; N, 10.29
Found: C, 50.96; H, 5.12; N, 10.66

3(RS)-[4-[(Trifluoromethylsulfonyl)amino]benzoyl]-L-valyl-N-[3-(1,1,1-trifluoro-4-methyl-2-oxopentyl)]-L-prolinamide (Formula Ib, $R^1$=$CH(CH_3)_2$, $R^2$=$CH(CH_3)_2$, $R^4$=H, A=$CF_3S(O_2)$NH- para to -CO-).

a. Ethyl 4-[(Trifluoromethylsulfonyl)amino]benzoate.
Trifluoromethanesulfonic anhydride (4.1 ml) was added dropwise to a precooled (0°) solution of ethyl p-aminobenzoate (3.3 g) in $CH_2Cl_2$ (50 ml) under nitrogen. The reaction mixture was stirred for 1 hr at 0°, then was allowed to warm to room temperature and was stirred for 1 hr. After the reaction mixture was concentrated under vacuum, EtOAc (125 ml) was added to the residue; and the resultant organic solution was washed (1N HCl, then brine), dried ($MgSO_4$), and concentrated under vacuum. The resulting residue was purified by flash chromatography on silica gel eluting with $CHCl_3$:MeOH (95:5) to give the product as a white powder (1.27 g); TLC, $R_f$=0.37, silica gel, $CHCl_3$:$Me_3OH$ (90:10).

b. 4-[(Trifluoromethylsulfonyl)amino]benzoic acid.
A solution of 1N NaOH (8.4 ml) was added to a stirred solution of the product of Example 10a (1.25 g) in MeOH (25 ml). Water (2 ml) was added, and the reaction mixture was stirred overnight. After the MeOH was distilled off under water aspiration vacuum, the resulting aqueous residue was diluted with water (20 ml). The aqueous solution was washed with EtOAc, made acidic (pH 2) with 1N HCl, and extracted with EtOAc (total=40 ml). The organic phase was dried ($MgSO_4$) and concentrated under vacuum to give the product as a white powder (1.05 g); TLC, $R_f$=0.4, silica gel, $CHCl_3$:MeOH:HOAc (96:4:0.2).

c. 2(RS),3(SR)-[4-[(Trifluoromethylsulfonyl)amino]benzoyl]-L-valyl-N-[3-(1,1,1-trifluoro-2-hydroxy-4-methylpentyl)]-L-prolinamide (Formula VIIb, $R^1 = CH(CH_3)_2$, $R^2 = CH(CH_3)_2$, $R^4 = H$, $A = CF_3S(O_2)NH$-para to -CO-).

WSCDI (0.74 g) was added to a stirred solution of the product of Example 10b (0.95 g), DMAP (0.473 g), the product of Example 2d (1.29 g), and DMF (4 ml) in dry $CH_2Cl_2$ (35 ml) at room temperature under nitrogen. The reaction mixture was stirred for 3 days. The reaction mixture was concentrated under vacuum. Ethyl acetate (75 ml) and 1N HCl (50 ml) were added to the resulting residue with stirring. The organic layer was separated, washed (1N HCl, then brine), dried ($MgSO_4$), and concentrated under vacuum. The resulting residue was purified by flash chromatography on acidic silica gel eluting with $CHCl_3$:MeOH (7.5:2.5) to give the product as an off-white dry foam (1.13 g); TLC, $R_f = 0.51$ and 0.57, silica gel, $CHCl_3$:MeOH:HOAc (95:5:0.2).

d. 3(RS)-[4-(Trifluoromethylsulfonyl)amino]benzoyl]-L-valyl-N-[3-(1,1,1-trifluoro-4-methyl-2-oxopentyl)]-L-prolinamide (Formula Ib, $R^1 = CH(CH_3)_2$, $R^2 = CH(CH_3)_2$, $R^4 = H$, $A = CF_3S(O_2)NH$- para to -CO-).

Acetic anhydride (6 ml) was added to a stirred solution of the product of Example 10c (0.54 g) in dry DMSO (6 ml) under nitrogen at room temperature. The reaction mixture was stirred at room temperature overnight. The mixture was poured into ice/water (100 ml) and stirred vigorously for one hour. The aqueous mixture was extracted with EtOAc (3 x 50 ml). The organic extracts were washed with saturated $NaHCO_3$ (2 x 15 ml) and brine, dried ($MgSO_4$), and concentrated under vacuum. The resulting residue was purified by flash chromatography on acidic silica gel eluting with $CHCl_3$, then $CHCl_3$:MeOH (95.5:1.5) to give the product as a white dry foam (0.235 g); TLC, $R_f = 0.43$ and 0.60, silica gel, $CHCl_3$:MeOH:HOAc (96:4:0.2); HPLC, $tr_R = 6.47$ and 9.42, Col B, FR = 3, $H_2O$:$CH_3CN$:THF:TFA (55:35:15:0.1).

Analysis calculated for:

$C_{24}H_{30}F_6N_4O_6S$. 1.0 $H_2O$: C,45.43; H, 5.08; N, 8.83

Found: C,45.57; H, 4.81; N, 8.69

Example 11

2S,3S-[(Phenylmethoxy)carbonyl]-L-valyl-N-[3-(1,1,1-trifluoro-2-hydroxy-4-methylpentyl)]-L-prolinamide.

a. 2R,3S-3-Amino-1,1,1,-trifluoro-4-methyl-2-pentanol D-tartaric acid salt.

Amine hydrochloride (20 g) generated as in Example 1c was dissolved in $H_2O$ and neutralized with solid $NaHCO_3$. The aqueous solution was extracted several times with $CH_2Cl_2$. The combined extracts were dried ($Na_2SO_4$) and concentrated to yield the amine frce base (14.04 g) as a white solid. This material was combined with D-tartaric acid (12.31 g) in boiling anhydrous EtOH (100 ml), and the resultant cloudy solution was filtered hot through filter paper. The solution was first cooled slowly to room temperature overnight and then placed in the refrigerator for several hours. Precipitate was collected on a fritted funnel, washed with cold EtOH, and dried overnight in a vacuum oven at 40°. A sample of the dried white solid (4.56 g) melted at 127°-130°. Most of this material (4.05 g) was dissolved in boiling EtOH (20 ml), and the solution was slowly cooled to room temperature. The white gel-like solid which deposited was collected in a sintered glass funnel and washed with several portions of EtOH. Vacuum oven drying at 40°C for several hours gave a white solid, mp 132°-134°C.

b. 2S,3S-[(Phenylmethoxy)carbonyl]-L-valyl-N-[3-(1,1,1-trifluoro-2-hydroxy-4-methylpentyl)]-L-prolinamide.

Acid prepared according to Example 2b (1.00 g, 2.87 mmol) was dissolved in dry THF (16 ml) under $N_2$ in a 50-ml, 3-necked, round-bottom flask equipped with a thermometer, $N_2$ inlet, septum and a magnetic stir bar. NMM (0.34 ml, 3.09 mmol) was added and the resultant stirred solution was cooled to an internal temperature of -35°. Isobutyl chloroformate (0.37 ml, 2.85 mmol) was added over 2 min, never allowing the internal temperature to rise above -35°. The reaction was stirred for 1 hr at -45° to -35°. The D-tartarate salt from Example 11a (0.92 g, 2.86 mmol) in a mixture of THF (5 ml) and DMSO (2 ml) was treated with NMM (0.68 ml) and the cloudy solution was added to the reaction mixture at such a rate that the temperature was kept below -40°. The reaction was stirred at -45° to -15° for 1 hr before it was allowed to warm to room temperature overnight. The mixture was diluted with $CHCl_3$ and then washed ($H_2O$, sat'd aq $NaHCO_3$), dried ($Na_2SO_4$), and concentrated to give the title product (1.15 g, 80%) as a white solid. The $^1H$-NMR spectrum of this material in DMSO-$d_6$ exhibited a doublet at $\delta 6.43$, which is the appropriate chemical shift of the alcohol proton in material with the assigned relative configuration.

This method provides substantially enantiomerically and diastereomerically pure material corresponding to one of the epimers of the mixture described in Example 2c.

Example 12

2(RS),3(SR)-N-[3-(1,1,1-trifluoro-2-hydroxy-4-methylpentyl)]-L-prolinamide (Formula IVa, $R^1 = CH(CH_3)CH_3$).

The title product was obtained without removal of the minor pair diastereomers as described in parts a-d of this example; the title product was obtained with the minor pair of diastereomers removed as described in parts e and f of this example.

a. 2(RS),3(SR)-4-Methyl-3-nitro-1,1,1-trifluoro-2-pentanol (Formula VI, $R^1=CH(CH_3)_2$).

See EXPLOSION WARNING before Example 1.

1-Nitro-2-methylpropane (37.7 g, 0.366 mol) from Example 1a, trifluoroacetaldehyde ethyl hemiacetal (58.5 g, 0.366 mol, 90% purity) and $K_2CO_3$ (3.4 g, 0.025 mol) were mixed and stirred at 60°C for 3 hr. and then at room temperature for 3 days. Brine (75 ml) and 1N aqueous HCl (50 ml) were added and the lower organic layer separated. The aqueous layer was extracted with $Et_2O$ (twice with 250 ml each) and the combined organic layers were washed with brine, dried over $Na_2SO_4$, filtered and concentrated under vacuum. The residue was purified by flash chromatography on silica gel with a gradient elution of $CH_2Cl_2$:hexane (50:50), $CH_2Cl_2$:hexane (75:25), $CH_2Cl_2$(100%) and MeOH:$CH_2Cl_2$ (5:95) to give the product (44.9 g); TLC, $R_f=0.65$, silica gel, EtOAc:$CHCl_3$ (5:95).

b. 2(RS,3(SR)-3-Amino-4-methyl-1,1,1-trifluoro-2-pentanol hydrochloride salt (Formula V, $R^1=CH(CH_3)_2$).

A solution of a portion of the product of Example 12a (37.0 g, 0.184 mol) in $Et_2O$ (200 ml) was added dropwise to a suspension of lithium aluminum hydride (22.0 g, 0.58 mol) in $Et_2O$ (800 ml). The reaction mixture was stirred for 45 min. and a saturated aqueous solution of $Na_2SO_4$ (110 ml) was carefully added. The resulting suspension was filtered; the filtrate was treated with ethereal HCl and concentrated under vacuum to give the product (37.6 g) which was used without further purification. $^1H$ NMR data ($CD_3COCD_3$)(250 MHz): 1.2δ, m, 6H; 2.3δ, m, 1H; 3.58δ, m, 1H; 4.98δ, m, 2H; 7.78δ, m, ($NH_2$).

c. 2(RS),3(SR)-1-[(Phenylmethoxy)carbonyl]-N-[3-(4-methyl-1,1,1-trifluoro-2-hydroxypentyl)]-L-prolinamide.

A solution of isobutyl chloroformate (11.01 g, 0.08 mol) in dry THF (30 ml) was added dropwise over 5 min to a pre-cooled solution (-15°C) of CBZ-L-proline (19.21 g, 0.077 mol) and N-methylmorpholine (8.18 g, 0.081 mol) in THF (300 ml) under a nitrogen atmosphere. The reaction mixture was stirred at -15° for 15 min. The reaction temperature was then reduced to -40°C and a solution of a portion of the product of Example 12b (16.00 g, 0.077 mol) and N-methylmorpholine (8.18 g, 0.081 mol) in THF (200 ml) was added dropwise to the reaction. The reaction mixture was stirred at -40°C for 1 hr and then allowed gradually to warm to room temperature and stirred for an additional hour. The reaction was filtered and concentrated under vacuum. The resulting syrup was dissolved in $CHCl_3$, and washed with aqueous 20% citric acid (twice with 75 ml each). The organic layer was concentrated under vacuum to give the crude product as a white cloudy syrup. The crude product was triturated with ether:hexane (1:2) to give 3 crops of the product as a white powder (17.11 g); TLC, $R_f=0.47$, silica gel, MeOH:$CHCl_3$ (3:97); m.p., 152°-154°C; HPLC, $t_R$ = 14.06, 16.63, 18.23, 19.00, Zorbax® ODS analytical column, $H_2O$:$CH_3CN$ (70:30), flow rate = 3 ml/min.

d. 2(RS),3(SR)-N-[3-(4-Methyl-1,1,1-trifluoro-2-hydroxypentyl)]-L-prolinamide (Formula IVa, $R^1=CH(CH_3)_2$).

The product of Example 12c (2.00 g, 4.97 mmol) was dissolved in absolute ethanol (50 ml), 10% Pd/C (0.5 g) was added and the reaction mixture was hydrogenolyzed (310126.53 Pascals, 45 psi hydrogen) for 3 hr at room temperature. The reaction mixture was filtered through Celite® and the solvent was removed under vacuum to give the product (1.36 g) which was used without further purification.

e. 2(RS),3(SR)-1-Phenylmethoxycarbonyl-N-[3-(1,1,1-trifluoro-2-hydroxy-4-methylpentyl)]-L-prolinamide.

Using the method of Example 12c, material prepared according to the method of Example 1 was allowed to react with CBZ-L-proline to provide the title compound (100%); TLC, $R_f=0.37$ & 0.45, MeOH: $CH_2Cl_2$ (5:95).

f. 2(RS),3(SR)-N-[3-(1,1,1-trifluoro-2-hydroxy-4-methylpentyl)]-L-prolinamide (Formula IVa, $R^1=CH(CH_3)CH_3$).

Using the method of Example 12b, the product of Example 12e was converted into the title product (100%); TLC, $R_f=0.73$ & 0.81, MeOH:$CH_2Cl_2$ satd with $NH_4OH$ (15:85).

Example 13

2(RS),3(SR)-L-α-aminobutyryl-N-[3-(1,1,1-trifluoro-2-hydroxy-4-methylpentyl)]-L-prolinamide trifluoroacetic acid salt (Formula IVb, $R^1=CH(CH_3)CH_3$, $R^2=CH_2CH_3$, $R^4=H$).

a. 2(RS),3(SR)-(1,1-Dimethylethoxycarbonyl)-L-α-aminobutyryl-N-[3-(1,1,1-trifluoro-2-hydroxy-4-methylpentyl)]-L-prolinamide.

DCC (5.90 g, 28.7 mmol) was added to a stirred solution of HOBT (7.76 g, 57.4 mmol), BOC-α-aminobutanoic acid (5.60 g, 27.4 mmol), and material prepared by the procedure of Example 12d (7.00 g, 26.1 mmol) in dry THF (130 ml) at 0° under nitrogen. After the resulting reaction mixture had been stirred at 0° for 1 hr, it was allowed to warm to room temperature and was stirred overnight. The reaction mixture was filtered; and the filtrate was concentrated under vacuum to a residue which was redissolved in EtOAc. The resulting solution was washed satd $NaHCO_3$ brine), dried ($Na_2SO_4$), filtered, and concentrated under vacuum to a residue which was purified by flash chromatography (EtOAc:$CH_2Cl_2$(1:3)) to give the title compound (95%); TLC, $R_f=0.29$, EtOAc:$CH_2Cl_2$ (3:7).

b. 2(RS),3(SR)-L-α-aminobutyryl-N-[3-(1,1,1-trifluoro-2-hydroxy-4-methylpentyl)]-L-prolinamide trifluoroacetic acid salt (Formula IVb, $R^1=CH(CH_3)CH_3$, $R^2=CH_2CH_3$, $R^4=H$).

A solution of material prepared by the procedure of Example 13a (4.0 g, 8.84 mmol) and TFA (32 ml, 415 mmol) in $CH_2Cl_2$ (32 ml) was stirred at room temperature for 22 hr before the solvents were removed under reduced pressure to afford the crude product (5 g, >100%) as a colorless glass which was used without further purification or characterization.

Example 14

2(RS),3(SR)-$N^6$-Phenylmethoxycarbonyl-L-lysyl-N-[3-(1,1,1-trifluoro-4-methyl-2-oxopentyl)]-L-prolinamide trifluoroacetic acid salt (Formula IVb, $R^1=CH(CH_3)CH_3$, $R^2=ØCH_2OCONH(CH_2)_4$-, $R^4=H$).

a. 2(RS),3(SR)-$N^2$-(1,1-Diemthylethoxycarbonyl)-$N^6$-phenylmethoxycarbonyl-L-lysyl-N-[3-(1,1,1-trifluoro-2-hydroxy-4-methylpentyl)]-L-prolinamide.

Using the method of Example 8b, $N^2$-BOC-$N^6$-CBZ-L-lysine was allowed to react with material prepared by the method of Example 12d to provide, after purification by flash chromatography (gradient elution, MeOH:$CHCl_3$ (2.5:97.5) to (5:95)), the title product (73%); TLC, $R_f=0.57$, MeOH:$CHCl_3$ (5:95).

b. 2(RS),3(SR)-$N^6$-Phenylmethoxycarbonyl-L-lysyl-N-[3-(1,1,1-trifluoro-4-methyl-2-oxopentyl)]-L-prolinamide trifluoroacetic acid salt (Formula IVb, $R^1=CH(CH_3CH_3)$, $R^2=ØCH_2OCONH(CH_2)_4$-, $R^4=H$).

To a solution of product prepared according to the procedure of Example 14a (2.75 g, 4.4 mmol) in $CH_2Cl_2$ (7 ml) was added TFA (10.4 g, 90 mmol), and the solution was stirred at room temperature for 1 hr. Toluene (10 ml) was added, and the reaction mixture was concentrated under vacuum to afford the title product (3.4 g) which was used without further purification.

Example 15

2(RS),3(SR)-L-Phenylalanyl-N-[3-(1,1,1-trifluoro-2-hydroxy-4-methylpentyl)]-L-prolinamide (Formula IVb, $R^1=CH(CH_3)CH_3$, $R^2=CH_2Ø$, $R^4=H$).

a. 2(RS),3(SR)-Phenylmethoxycarbonyl-L-phenylalanyl-N-[3-(1,1,1-trifluoro-2-hydroxy-4-methylpentyl)]-L-prolinamide.

After DCC (2.27 g, 11.0 mmol) was added to a stirred solution of CBZ-L-phenylalanine (2.29 g, 10.0 mmol), HOBT (2.70 g, 17.6 mmol) and material prepared according to Example 12f (2.68 g, 10.0 mmol) in dry THF (45 ml) chilled to -17°C, the mixture was left at room temperature overnight, filtered, and concentrated to remove the THF before it was taken up in $Et_2O$ and EtOAc. The resulting solution was washed (satd $NaHCO_3$ (2x), 1N HCl, brine), dried ($MgSO_4$), filtered, concentrated, and taken up in minmal $CH_2Cl_2$. After N,N'-dicyclohexylurea was filtered, the solution was concentrated under vacuum, and dried under vacuum to provide the title compound in quantitative yield as a white foam; TLC, $R_f=$ 0.37 & 0.45, MeOH:$CH_2Cl_2$(5:95).

b. 2(RS),3(SR)-L-Phenylalanyl-N-[3-(1,1,1-trifluoro-2-hydroxy-4-methylpentyl)]-L-prolinamide (Formula IVb, $R^1=CH(CH_3)CH_3$, $R^2=CH_2Ø$, $R^4=H$).

A mixture of the product of Example 15a (501.3 mg, 0.898 mmol) and 50%-water wet 10% Pd/C (50 mg) in absolute EtOH (17 ml) was stirred uner hydrogen (1 atmosphere, 101,325 Pascals) overnight, filtered, concentrated and dried under vacuum to provide the title compound in quantitative yield; TLC, $R_f=0.14$, MeOH: $CH_2Cl_2$ (5:95); $R_f=0.43$ & 0.48 MeOH: $NH_4OH$ satd $CH_2Cl_2$(5:95).

Example 16

2(RS),3(SR)-L-Phenylglycyl-N-[3-(1,1,1-trifluoro-2-hydroxy-4-methylpentyl)]-L-prolinamide (Formula IVb, $R^1=CH(CH_3)CH_3$, $R^2=Ø$, $R^4=H$).

a. 2(RS),3(SR)-Phenylmethoxycarbonyl-L-phenylglycyl N-[3-(1,1,1-trifluoro-2-hydroxy-4-methylpentyl)]-L-prolinamide.

Using a method similar to the method of Example 8b, except using $CHCl_3$ instead of EtOAc to dissolve the residue, washing the $CHCl_3$ solution with 20% citric acid solution and drying it over $Na_2O_4$, material prepared according to the procedure of Example 12f was allowed to react with CBZ-L-phenylglycine to provide, after purification by flash chromatography (MeOH:$CH_2Cl_2$(5:95)), the title product (95%); TLC, $R_f=0.13$ & 0.19, MeOH:$CH_2Cl_2$ (5:95).

b. 2(RS),3(SR)-L-Phenylglycyl-N-[3-(1,1,1-trifluoro-2-hydroxy-4-methylpentyl)]-L-prolinamide (Formula IVb, $R^1=CH(CH_3)CH_3$, $R^2=Ø$, $R^4=H$).

Using the method of Example 2d, material prepared according to the procedure of Example 16a was converted into the title product in 100% yield.

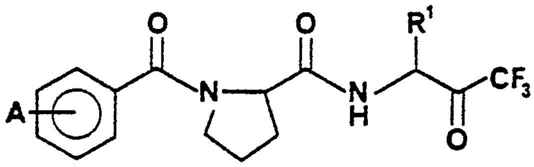

Ia

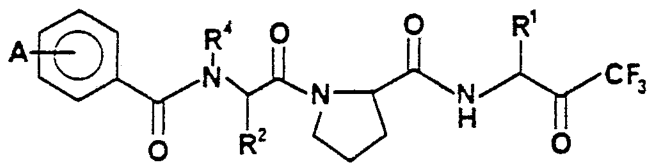

Ib

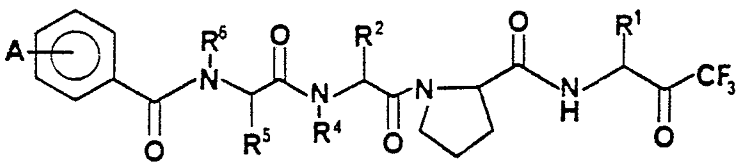

Ic

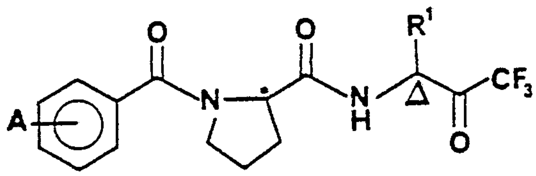

IIa

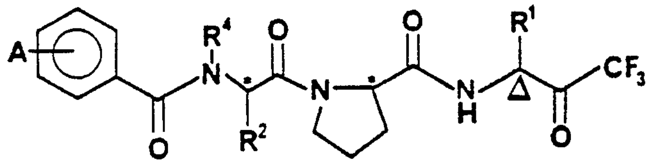

IIb

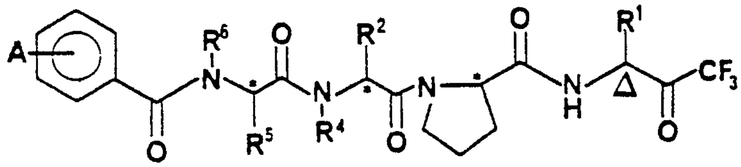

IIc

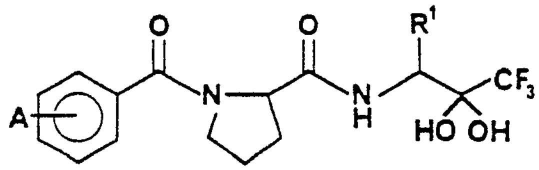

IIIa

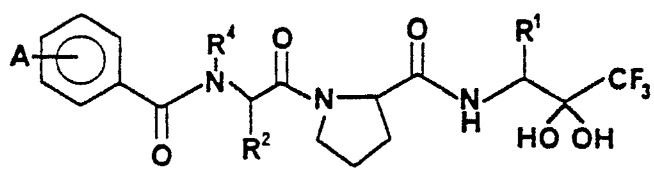

IIIb

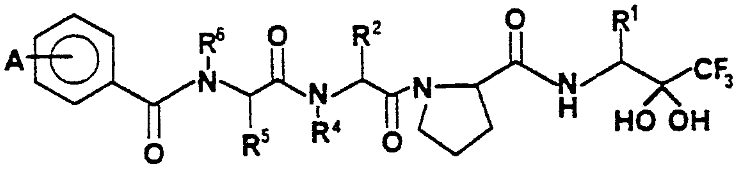

IIIc

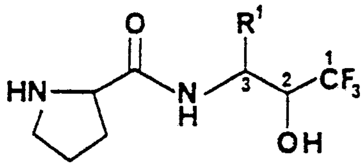

IVa

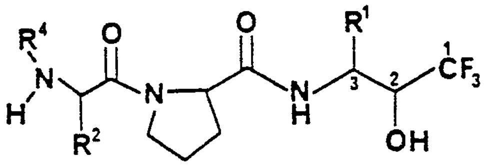

IVb

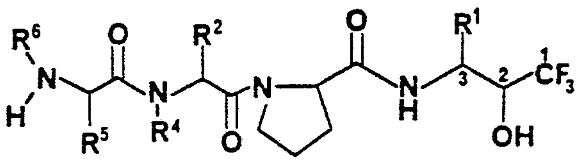

IVc

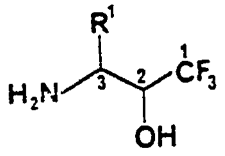

V

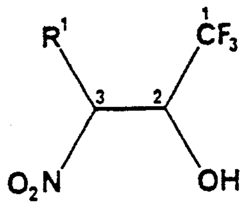

VI

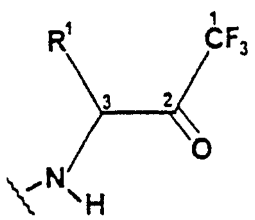

I, II & X
(portion)

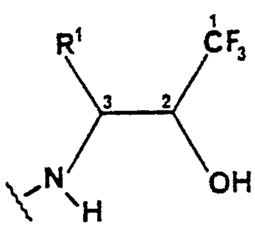

IV, VII & IX
(portion)

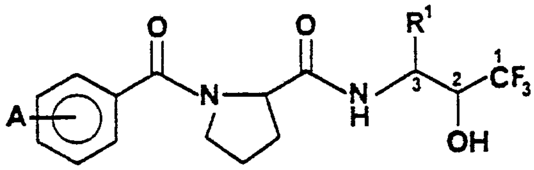

VIIa

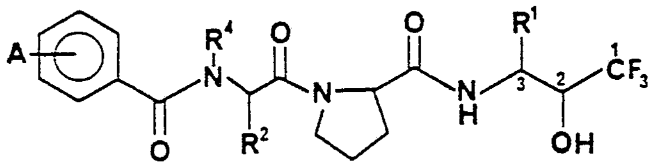

VIIb

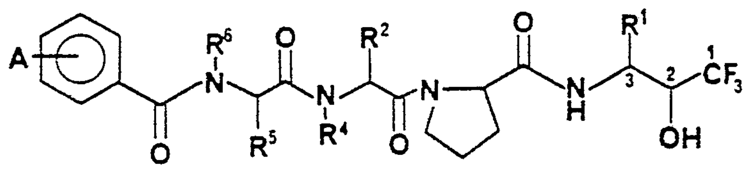

VIIc

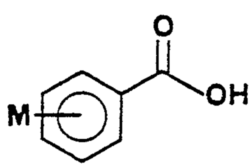

VIII

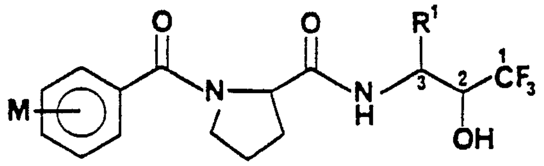

IXa

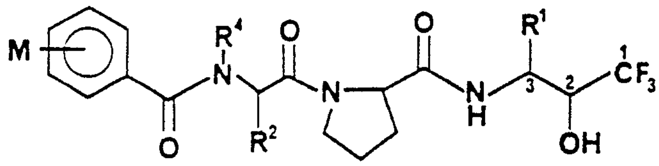

IXb

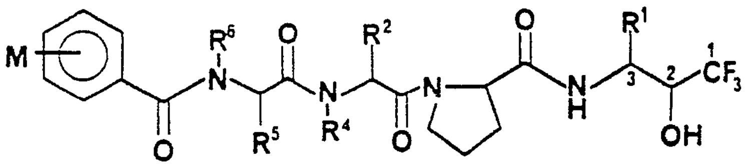

IXc

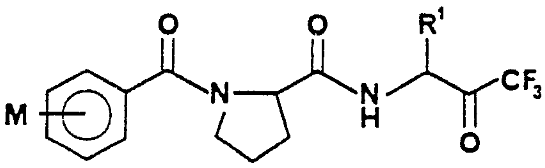

Xa

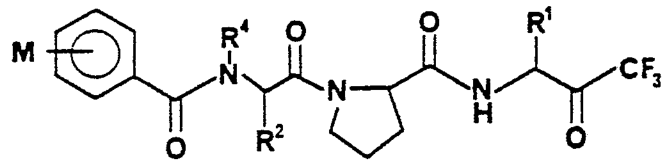

Xb

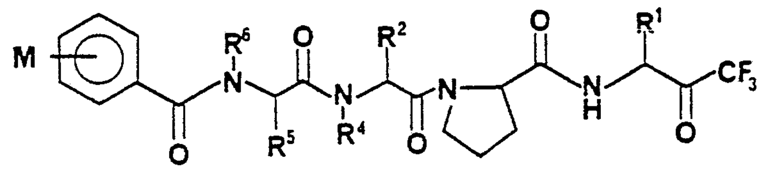

Xc

## Claims

1. A compound of the following formula Ia, Ib or Ic set out hereinbelow wherein
$R^1$ is an alkyl group containing from 1 to 5 carbon atoms;
$R^2$ and $R^5$ are each selected independently from a group consisting of:
(I) an alkyl group containing from 1 to 10 carbons;
(II) an alkyl group containing from 1 to 6 carbons substituted by at least one member selected from a group consisting of:
(a) hydroxy;
(b) amino;
(c) alkylamino containing from 1 to 6 carbons;
(d) dialkylamino wherein each alkyl group contains from 1 to 6 carbons;
(e) alkanoyl containing from 1 to 6 carbons;
(f) arylcarbonyl wherein the aryl contains 6, 10 or 12 carbons;
(g) aralkanoyl containing 8 to 13 carbons;
(h) amido which may be attached to the alkyl group via either a nitrogen or carbon of said amido;
(i) alkylcarbonylamino wherein the alkyl group contains from 1 to 6 carbons;
(j) alkylaminocarbonyl wherein the alkyl group contains from 1 to 6 carbons;
(k) arylcarbonylamino wherein the aryl group contains 6, 10 or 12 carbons;
(l) aralkylcarbonylamino wherein the aralkyl group contains from 7 to 13 carbons;
(m) arylaminocarbonyl wherein the aryl group contains 6, 10 or 12 carbons;
(n) aralkylaminocarbonyl wherein the aralkyl group contains from 7 to 13 carbons;
(o) carboxy;
(p) aryloxycarbonyl wherein the aryl group contains 6, 10 or 12 carbons;
(q) aralkoxycarbonyl wherein the aralkoxy group contains from 7 to 13 carbons;
(r) alkanoyloxy containing from 1 to 6 carbons;
(s) aroyloxy wherein the aryl portion contains 6, 10 or 12 carbons;
(t) aralkanoyloxy containing from 8 to 14 carbons;
(u) alkylsulfonamido wherein the alkyl group contains from 1 to 6 carbons;
(v) aralkylsulfonamido wherein the aralkyl group contains from 7 to 13 carbons;
(w) arylsulfonamido wherein the aryl group contains 6, 10 or 12 carbons;
(x) acylsulfonamido containing 1 to 15 carbons, including acylsulfon amido wherein the acyl group contains 1 to 7 carbons when it is the terminal portion of the acylsulfonamide and provided that when the acylsulfonamido contains an aryl the aryl may be further substituted by a member selected from fluoro, chloro, bromo, iodo and nitro;
(y) alkoxycarbonyl wherein the alkoxy group contains from 1 to 6 carbons;
(z) aralkoxycarbonylamino containing from 8 to 13 carbons;
(aa) aryloxycarbonylamino wherein the aryloxy group contains 6, 10 or 12 carbons;
(bb) alkoxycarbonylamino wherein the alkyloxy group contains from 1 to 6 carbons;
(cc) aryl containing 6, 10 or 12 carbons;

(dd) aryl containing 6, 10 or 12 carbons and substituted by 1 to 3 members selected from a group consisting of chloro, bromo, iodo, fluoro, trifluoromethyl, hydroxy, alkyl (1 to 6 carbons), alkoxy (1 to 6 carbons), alkoxycarbonyl (1 to 6 carbons), carboxy, 5-tetrazolo, and acylsufonamido (1 to 15 carbons) and provided that when the acylsulfonamido contains an aryl the aryl may be further substituted by a member selected from fluoro, chloro, bromo, iodo and nitro;
(ee) cycloalkyl containing from 3 to 15 carbons;
(ff) alkylureido wherein the alkyl group contains from 1 to 6 carbons;
(gg) aralkylureido containing from 8 to 13 carbons; and
(hh) arylureido wherein the aryl group contains 6, 10 or 12 carbons; and (III) phenyl;
$R^4$ and $R^6$ are each independently hydrogen or methyl; and
A is an acidic radical selected from a group consisting of a carbamoylsulfonamido radical of formula $R^7$.NH.CO.NH.S($O_2$)-, a sulfonylureido radical of formula $R^9$.S($O_2$).NH.CO.$NR^8$-, a heterocyclic acylsulfonamido radical of formula Q.B-, and a trifluoromethylsulfonamido radical of formula $CF_3$.S($O_2$).NH-wherein
$R^7$ is selected from a group consisting of:
(I) an alkyl group containing from 1 to 10 carbons;
(II) a cycloalkyl group containing from 3 to 10 carbons;
(III) an arylmethyl group containing 7 to 11 carbons; wherein the aryl group is optionally substituted of fluoro, chloro, bromo, iodo, trifluoromethyl, nitro, an alkyl group of 1 to 4 carbons, methoxy, ethoxy, and methylthio;
(IV) an aryl group containing 6 or 10 carbons and optionally substituted by 1 to 3 members of the group consisting of fluoro, chloro, bromo, iodo, trifluoromethyl, nitro, an alkyl group of 1 to 4 carbons, methoxy, ethoxy, and methylthio;
(V) an aromatic heterocyclic group as defined below for Q;
$R^8$ is hydrogen or methyl;
$R^9$ is selected from a group consisting of:
(I) an alkyl group containing from 1 to 10 carbons;
(II) a cycloalkyl group containing from 3 to 10 carbons;
(III) an aryl group containing 6 or 10 carbons and optionally substituted by 1 to 3 members of the group consisting of fluoro, chloro, bromo, iodo, trifluormethyl, methyl, nitro, hydroxy, carboxy, amino, dimethylamino, phenyl, and an alkylcarbonylamino group wherein the alkyl group contains 1 to 4 carbons;
(IV) an aromatic heterocyclic group as defined below for Q;
(V) a 2-phenylethyl group; and
(VI) a 2,2,2-trifluoroethyl group;
Q is an aromatic heterocyclic group of from 1 to 15 carbons and from 1 to 4 heteroatoms each of which is selected independently from a group consisting of sulfur, nitrogen and oxygen, and which form 1 to 3 five or six-membered rings at least one of which is aromatic, and optionally, wherein up to 3 carbons of the aromatic ring(s) may be substituted at any carbon atom with a member of a group consisting of fluoro, chloro, bromo, iodo and trifluoromethyl, and provided further than any nitrogen may be substituted by an alkyl group containing from 1 to 6 carbons; and
B is an acylsulfonamide linkage selected from a group consisting of those represented by formulae (i) -S($O_2$NHCO- and (ii) -CONHS($O_2$)-; or,
where appropriate, a pharmaceutically acceptable acid- or base-addition salt thereof.

2. A compound as claimed in Claim 1 selected from a group consisting of:
(i) a compound wherein A is a carbamoylsulfonamido radical of formula $R^7$.NH.CO.NH.S($O_2$)-,
(ii) a compound wherein A is a sulfonylureido radical of formula $R^9$.S($O_2$).NH.CO.$NR^8$-,
(iii) a compound wherein A is a heterocyclic acylsulfonamido radical of formula Q.B-, and
(iv) a compound wherein A is a trifluoromethylsulfonamido radical of formula $CF_3$.S($O_2$).NH-.

3. A compound as claimed in Claim 1 or 2 wherein:
$R^1$ is methyl, ethyl, n-propyl, iso-propyl, n-butyl, sec-butyl, iso-butyl, n-pentyl, pent-2-yl, pent-3-yl, 2-methylbutyl, or 3-methylbutyl;
$R^2$ and $R^5$ are each selected independently from a group consisting of:
(I) methyl, ethyl, n-propyl, isopropyl, n-butyl, sec-butyl, iso-butyl, n-pentyl, pent-2-yl, pent-3-yl, 2-methylbutyl, 3-methylbutyl, hexyl, heptyl, octyl, nonyl, and decyl;
(II) methyl, ethyl, n-propyl, isopropyl, n-butyl, sec-butyl, iso-butyl, n-pentyl, pent-2-yl, pent-3-yl, 2-methylbutyl, 3-methylbutyl, and hexyl substituted by at least one member selected from a group consisting of:
(a) hydroxy;
(b) amino;
(c) alkylamino containing from 1 to 6 carbons;
(d) dialkylamino wherein each alkyl group contains from 1 to 6 carbons;
(e) alkanoyl containing from 1 to 6 carbons;
(f) arylcarbonyl wherein the aryl contains 6, 10 or 12 carbons;
(g) aralkanoyl containing 8 to 13 carbons;
(h) amido which may be attached to the alkyl group via either a nitrogen or carbon of said amido;

(i) alkylcarbonylamino wherein the alkyl group contains from 1 to 6 carbons;
(j) alkylaminocarbonyl wherein the alkyl group contains from 1 to 6 carbons;
(k) arylcarbonylamino wherein the aryl group contains 6, 10 or 12 carbons;
(l) aralkylcarbonylamino wherein the aralkyl group contains from 7 to 13 carbons;
(m) arylaminocarbonyl wherein the aryl group contains 6, 10 or 12 carbons;
(n) aralkylaminocarbonyl wherein the aralkyl group contains from 7 to 13 carbons;
(o) carboxy;
(p) aryloxycarbonyl wherein the aryl group contains 6, 10 or 12 carbons;
(q) aralkoxycarbonyl wherein the aralkoxy group contains from 7 to 13 carbons;
(r) alkanoyloxy containing from 1 to 6 carbons;
(s) aroyloxy wherein the aryl portion contains 6, 10 or 12 carbons;
(t) aralkanoyloxy containing from 8 to 14 carbons;
(u) alkylsulfonamido wherein the alkyl group contains from 1 to 6 carbons;
(v) aralkylsulfonamido wherein the aralkyl group contains from 7 to 13 carbons;
(w) arylsulfonamido wherein the aryl group contains 6, 10 or 12 carbons;
(x) acylsulfonamido (1 to 15 carbons) including acylsulfonamido wherein the acyl group contains 1 to 7 carbons when it is the terminal portion of the acylsulfonamide and provided that when the acylsulfonamido contains an aryl the aryl may be further substituted by a member selected from fluoro, chloro, bromo, iodo and nitro;
(y) alkoxycarbonyl wherein the alkoxy group contains from 1 to 6 carbons;
(z) benzyloxycarbonyl amino;
(aa) aryloxycarbonylamino wherein the aryloxy group contains 6, 10 or 12 carbons;
(bb) alkoxycarbonylamino wherein the alkyloxy group contains from 1 to 16 carbons;
(cc) phenyl, biphenyl, and naphthyl;
(dd) aryl containing 6, 10 or 12 carbons and substituted by 1 to 3 members selected from a group consisting of chloro, bromo, iodo, fluoro, trifluoromethyl, hydroxy, alkyl (1 to 6 carbons), alkoxy (1 to 6 carbons), alkoxycarbonyl (1 to 6 carbons), carboxy, 5-tetrazolo, and acylsufonamido (1 to 15 carbons) and provided that when the acylsulfonamido contains an aryl the aryl may be further substituted by a member selected from fluoro, chloro, bromo, iodo and nitro;
(ee) cyclohexyl, adamantyl, and norbornyl;
(ff) alkylureido wherein the alkyl group contains from 1 to 6 carbons;
(gg) aralkylureido containing from 8 to 13 carbons; and
(hh) arylureido wherein the aryl group contains 6, 10 or 12 carbons; and (III) phenyl;
$R^4$ and $R^6$ are each independently hydrogen or methyl; and
A is an acidic radical selected from a group consisting of a carbamoylsulfonamido radical of formula $R^7$.NH.CO.NH.S($O_2$)-, a sulfonylureido radical of formula $R^9$.S($O_2$).NH.CO.$NR^8$-, a heterocyclic acylsulfonamido radical of formula Q.B-, and a trifluoromethylsulfonamido radical of formula $CF_3$.S($O_2$).NH-wherein
$R^7$ is selected from a group consisting of:
(I) an alkyl group as listed above for an alkyl group of $R^2$ or $R^5$;
(II) cyclopropyl, cyclobutyl, cyclopentyl, cyclohexyl, norbornyl, and adamantyl;
(III) benzyl and naphthylmethyl wherein the aryl group is optionally substituted by 1 to 3 members of the group consisting of fluoro, chloro, bromo, iodo, trifluoromethyl, nitro, an alkyl group of 1 to 4 carbons, methoxy, ethoxy, and methylthio;
(IV) phenyl and naphthyl and optionally substituted by 1 to 3 members of the group consisting of fluoro, chloro, bromo, iodo, trifluoromethyl, nitro, an alkyl group of 1 to 4 carbons, methoxy, ethoxy, and methylthio;
(V) pyridyl, thienyl, furyl and quinolinyl;
$R^8$ is hydrogen or methyl;
$R^9$ is selected from a group consisting of:
(I) an alkyl group as listed above for an alkyl group of $R^2$ or $R^5$;
(II) cyclopropyl, cyclobutyl, cyclopentyl, cyclohexyl, norbornyl, and adamantyl;
(III) phenyl and naphthyl each of which optionally may be substituted by 1 to 3 members of the group consisting of fluoro, chloro, bromo, iodo, trifluormethyl, methyl, nitro, hydroxy, carboxy, amino, dimethylamino, phenyl, and an alkylcarbonylamino group wherein the alkyl group contains 1 to 4 carbons;
(IV) pyridyl, thienyl, furyl, and quinolinyl,
(V) a 2-phenylethyl group; and
(VI) a 2,2,2-trifluoroethyl group; and
Q is pyridyl, thienyl, furyl, or quinolinyl, optionally substituted at any carbon atom with a member of a group consisting of fluoro, chloro, bromo, iodo and trifluoromethyl.

4. A compound as claimed in Claim 1 or 2 wherein:
$R^1$ is isopropyl;
$R^2$ is selected from a group consisting of:
(I) an alkyl group containing 2 to 3 carbons;
(II)(q) ethyl substituted by aralkoxycarbonyl wherein the aralkoxy group contains 7 carbons;

(w) butyl substituted by an arylsulfonamido wherein the aryl portion has 6 carbons;
(x) ethyl substituted by acylsulfonamido containing 7 carbons;
(z) butyl substituted by aralkyloxycarbonylamino wherein the aralkoxy portion contains 7 carbons; and
(cc) methyl substituted by an aryl containing 6 carbons; and
(III) phenyl;
$R^4$, $R^6$ and $R^8$ are each selected as hydrogen;
and $R^5$ is selected from a group consisting of:
(I) n-butyl;
(II)(q) ethyl substituted by aralkoxycarbonyl wherein the aralkoxy contains 7 carbons; and
(II)(z) butyl substituted by aralkyloxycarbonylamino wherein the aralkyl group contains 7 carbons.

5. A compound as claimed in Claim 1 or 2 which is a compound represented by formula Ib, wherein $R^1$ and $R^2$ are isopropyl, $R^4$ is hydrogen and the radical A is para to the carbonyl group.

6. A compound as claimed in any one of Claims 1, 2, 3, 4 and 5 wherein:
$R^7$ is phenyl, 4-chlorophenyl or 1-naphthyl;
$R^8$ is hydrogen;
$R^9$ is phenyl or 4-chlorophenyl;
Q is 2-pyridyl; and
B is a linkage of formula $-S(O_2).NH.CO-$.

7. A compound as claimed in Claim 1 selected from

(i) 3(RS)-[4-[(N′-phenylureido)sulfonyl]benzoyl]-L-valyl-N-[3-(1,1,1,-trifluoro-4-methyl-2-oxopentyl)]-L-prolinamide,

(ii) 3(RS)-[4-[[N′-(4-chlorophenyl)ureido]sulfonyl]benzoyl]-L-valyl-N-[3-(1,1,1-trifluoro-4-methyl-2-oxopent-yl)]-L-prolinamide,

(iii) 3(RS)-[4-[[N′-(1-naphthalenyl)ureido]sulfonyl]benzoyl]-L-valyl-N-[3-(1,1,1-trifluoro-4-methyl-2-oxopentyl)]-L-prolinamide,

(ic) 3(RS)-[4-[N′-(phenylsulfonyl)ureido]benzoyl]-L-valyl-N-[3-(1,1,1-trifluoro-4-methyl-2-oxopentyl)]-L-prolinamide,

(v) 3(RS)-[4-[N′-[(4-chlorophenyl)sulfonyl]ureido]benzoyl]-L-valyl-N-[3-(1,1,1-trifluoro-4-methyl-2-oxopentyl)]-L-prolinamide,

(vi) 3(RS)-[4-[(2-pyridylsulfonylamino)carbonyl]benzoyl-L-valyl-N-[3-(1,1,1-trifluoro-4-methyl-2-oxopentyl)]-L-prolinamide, and

(vii) 3(RS)-[4-[(trifluoromethylsulfonyl)amino]benzoyl]-L-valyl-N-[3-(1,1,1-trifluoro-4-methyl-2-oxopentyl)]-L-prolinamide.

8. A salt as claimed in any preceeding claim wherein said salt is derived from a base forming a physiologically acceptable cation.

9. A method of making a compound of formulae Ia, Ib or Ic, set out hereinbelow, or a pharmaceutically acceptable salt thereof, as claimed in any one of Claims 1-8 which is characterized by:
(A) for a compound of formula Ia, Ib or Ic, oxidizing a corresponding hydroxy compound of formula VIIa, VIIb or VIIc;
(B) for a compound of formula Ia, Ib or Ic wherein A is a radical of formula $R^7.NH.CO.NH.S(O_2)-$, treating a corresponding compound of formula Xa, Xb or Xc wherein M is an aminosulfonyl radical of formula $H_2NS(O_2)-$ with an isocyanate of formula $R^7.NCO$;
(C) for a compound of formula Ia, Ib or Ic wherein A is a radical of formula $R^9.S(O_2).NH.CO.NR^8-$, treating a corresponding compound of formula Xa, Xb or Xc wherein M is an amino radical of formula $HNR^8-$ with a sulfonylisocyanate of formula $R^9.S(O_2).NCO$;
(D) for a compound of formula Ia, Ib or Ic wherein A is a radical of formula $R^9.S(O_2).NH.CO.NR^8-$and wherein $R^8$ is H, treating a corresponding compound of formula Xa, Xb or Xc wherein M is an isocyanate radical of formula ONC- with a sulfonamide of formula $R^9.S(O_2).NH_2$;
(E) for a compound of formula Ia, Ib or Ic wherein A is a radical of formula Q.B- in which -B- is $-S(O_2)NHCO-$, coupling a corresponding acid of formula Xa, Xb or Xc wherein M is COOH with a sulfonamide of formula $Q.S(O_2).NH_2$;
(F) for a compound of formula Ia, Ib or Ic wherein A is a radical of formula Q.B- in which -B- is $-CO.NH.S(O)_2)-$, coupling a corresponding sulfonamide of formula Xa, Xb or Xc wherein M is $H_2N.S(O_2)-$ with a heterocyclic acid of formula Q.COOH; and
(G) for a compound of formula Ia, Ib or Ic wherein A is a radical of formula $CF_3.S(O_2).NH-$, treating a corresponding amine of formula Xa, Xb or Xc wherein M is $H_2N-$ with trifluoromethanesulfonic anhydride;
wherein the chemical formulae VIIa, VIIb, VIIc, Xa, Xb and Xc are set out hereinbelow; and wherein $R^1$, $R^2$, $R^4$-$R^9$, and A, except where more particularly described, have the meanings defined in any one of Claims 1-8;
and whereafter, when a pharmaceutically acceptable salt is required, reacting an acid form of the compound of formula Ia, Ib or Ic with a base affording a physiologically acceptable cation or reacting a suitably basic compound of formula Ia, Ib or Ic with an acid affording a physiologically acceptable anion.

10. A compound of formula VIIa, VIIb or VIIc wherein $R^1$, $R^2$, $R^4$-$R^9$ and A are as defined in Claim 1, or a salt thereof.

11. A compound of formula Xa, Xb or Xc wherein M is selected from a group consisting of $H_2NS(O_2)-$,

HNR$^8$-, and ONC and wherein $R^1$, $R^2$, $R^4$-$R^9$ and A have any of the meanings defined in Claim 1, or a salt thereof.

12. A pharmaceutical composition comprising a compound of Claim 1 or a pharmaceutically acceptable salt thereof as claimed in any one of Claims 1-8 and a pharmaceutically acceptable diluent or carrier.

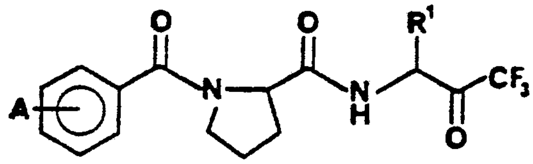

Ia

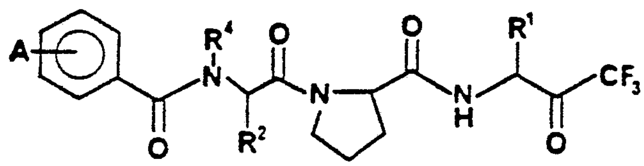

Ib

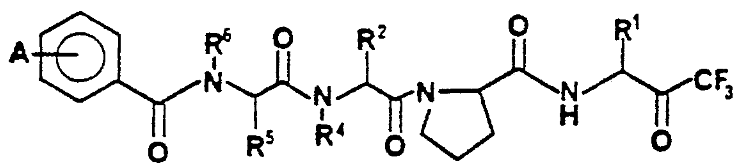

Ic

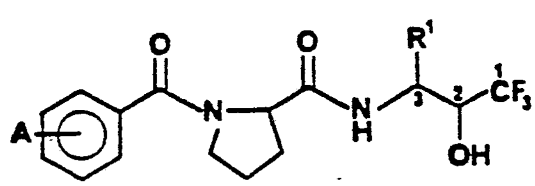

VIIa

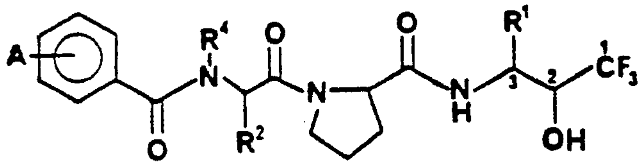

VIIb

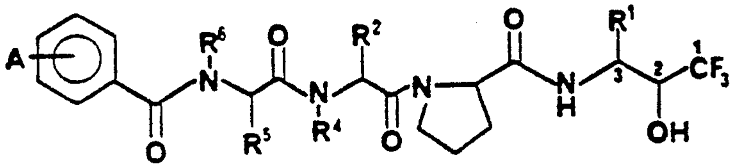

VIIc

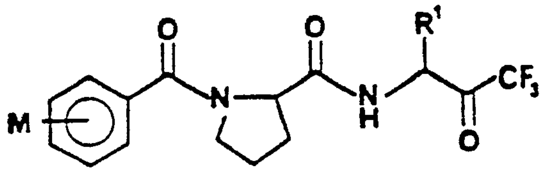

Xa

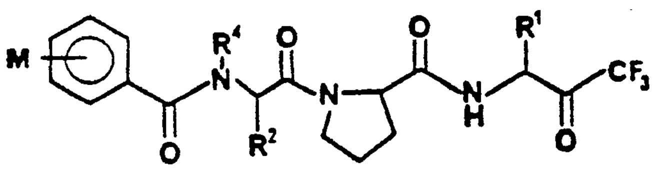

Xb

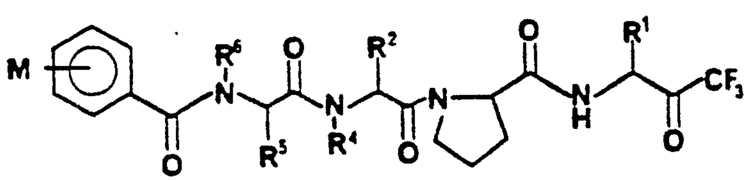

Xc

Claim for the following contracting state: ES

1. A process for producing a compound of the following formula Ia, Ib or Ic(as set out hereinbelow wherein

$R^1$ is an alkyl group containing from 1 to 5 carbon atoms;

$R^2$ and $R^5$ are each selected independently from a group consisting of:

(I) an alkyl group containing from 1 to 10 carbons;

(II) an alkyl group containing from 1 to 6 carbons substituted by at least one member selected from a group consisting of:

(a) hydroxy;

(b) amino;

(c) alkylamino containing from 1 to 6 carbons;

(d) dialkylamino wherein each alkyl group contains from 1 to 6 carbons;

(e) alkanoyl containing from 1 to 6 carbons;

(f) arylcarbonyl wherein the aryl contains 6, 10 or 12 carbons;

(g) aralkanoyl containing 8 to 13 carbons;

(h) amido which may be attached to the alkyl group via either a nitrogen or carbon of said amido;

(i) alkylcarbonylamino wherein the alkyl group contains from 1 to 6 carbons;

(j) alkylaminocarbonyl wherein the alkyl group contains from 1 to 6 carbons;

(k) arylcarbonylamino wherein the aryl group contains 6, 10 or 12 carbons;

(l) aralkylcarbonylamino wherein the aralkyl group contains from 7 to 13 carbons;

(m) arylaminocarbonyl wherein the aryl group contains 6, 10 or 12 carbons;

(n) aralkylaminocarbonyl wherein the aralkyl group contains from 7 to 13 carbons;

(o) carboxy;

(p) aryloxycarbonyl wherein the aryl group contains 6, 10 or 12 carbons;

(q) aralkoxycarbonyl wherein the aralkoxy group contains from 7 to 13 carbons;

(r) alkanoyloxy containing from 1 to 6 carbons;

(s) aroyloxy wherein the aryl portion contains 6, 10 or 12 carbons;

(t) aralkanoyloxy containing from 8 to 14 carbons;

(u) alkylsulfonamido wherein the alkyl group contains from 1 to 6 carbons;

(v) aralkylsulfonamido wherein the aralkyl group contains from 7 to 13 carbons;

(w) arylsulfonamido wherein the aryl group contains 6, 10 or 12 carbons;

(x) acylsulfonamido containing 1 to 15 carbons, including acylsulfonamido wherein the acyl group contains 1 to 7 carbons when it is the terminal portion of the acylsulfonamide and provided that when the acylsulfonamido contains an aryl the aryl may be further substituted by a member selected from fluoro, chloro, bromo, iodo and nitro;

(y) alkoxycarbonyl wherein the alkoxy group contains from 1 to 6 carbons;

(z) aralkoxycarbonylamino containing from 8 to 13 carbons;

(aa) aryloxycarbonylamino wherein the aryloxy group contains 6, 10 or 12 carbons;

(bb) alkoxycarbonylamino wherein the alkyloxy group contains from 1 to 6 carbons;

(cc) aryl containing 6, 10 or 12 carbons;

(dd) aryl containing 6, 10 or 12 carbons and substituted by 1 to 3 members selected from a group consisting of chloro, bromo, iodo, fluoro, trifluoromethyl, hydroxy, alkyl (1 to 6 carbons), alkoxy (1 to 6 carbons), alkoxycarbonyl (1 to 6 carbons), carboxy, 5-tetrazolo, and acylsufonamido (1 to 15 carbons) and provided that when the acylsulfonamido contains an aryl the aryl may be further substituted by a member selected from fluoro, chloro, bromo, iodo and nitro;

(ee) cycloalkyl containing from 3 to 15 carbons;

(ff) alkylureido wherein the alkyl group contains from 1 to 6 carbons;

(gg) aralkylureido containing from 8 to 13 carbons; and

(hh) arylureido wherein the aryl group contains 6, 10 or 12 carbons; and (III) phenyl;

$R^4$ and $R^6$ are each independently hydrogen or methyl; and

A is an acidic radical selected from a group consisting of a carbamoylsulfonamido radical of formula $R^7.NH.CO.NH.S(O_2)$-, a sulfonylureido radical of formula $R^9.S(O_2).NH.CO.NR^8$-, a heterocyclic acylsulfonamido radical of formula Q.B-, and a trifluoromethylsulfonamido radical of formula $CF_3.S(O_2).NH$-wherein

$R^7$ is selected from a group consisting of:

(I) an alkyl group containing from 1 to 10 carbons;

(II) a cycloalkyl group containing from 3 to 10 carbons;

(III) an arylmethyl group containing 7 to 11 carbons; wherein the aryl group is optionally substituted by 1 to 3 members of the group consisting of fluoro, chloro, bromo, iodo, trifluoromethyl, nitro, an alkyl group of 1 to 4 carbons, methoxy, ethoxy, and methylthio;

(IV) an aryl group containing 6 or 10 carbons and optionally substituted by 1 to 3 members of the group consisting of fluoro, chloro, bromo, iodo, trifluoromethyl, nitro, an alkyl group of 1 to 4 carbons, methoxy, ethoxy, and methylthio;
(V) an aromatic heterocyclic group as defined below for Q;
$R^8$ is hydrogen or methyl;
$R^9$ is selected from a group consisting of:
(I) an alkyl group containing from 1 to 10 carbons;
(II) a cycloalkyl group containing from 3 to 10 carbons;
(III) an aryl group containing 6 or 10 carbons and optionally substituted by 1 to 3 members of the group consisting of fluoro, chloro, bromo, iodo, trifluormethyl, methyl, nitro, hydroxy, carboxy, amino, dimethylamino, phenyl, and an alkylcarbonylamino group wherein the alkyl group contains 1 to 4 carbons;
(IV) an aromatic heterocyclic group as defined below for Q;
(V) a 2-phenylethyl group; and
(VI) a 2,2,2-trifluoroethyl group;
Q is an aromatic heterocyclic group of from 1 to 15 carbons and from 1 to 4 heteroatoms each of which is selected independently from a group consisting of sulfur, nitrogen and oxygen, and which form 1 to 3 five or six-membered rings at least one of which is aromatic, and optionally, wherein up to 3 carbons of the aromatic ring(s) may be substituted at any carbon atom with a member of a group consisting of fluoro, chloro, bromo, iodo and trifluoromethyl, and provided further that any nitrogen may be substituted by an alkyl group containing from 1 to 6 carbons; and
B is an acylsulfonamide linkage selected from a group consisting of those represented by formulae (i) $-S(O_2)NHCO-$ and (ii) $-CONHS(O_2)-$; or,
where appropriate, a pharmaceutically acceptable acid- or base-addition salt thereof, which method is selected from
(A) for a compound of formula Ia, Ib or Ic, oxidizing a corresponding hydroxy compound of formula VIIa, VIIb or VIIc;
(B) for a compound of formula Ia, Ib or Ic wherein A is a radical of formula $R^7.NH.CO.NH.S(O_2)-$, treating a corresponding compound of formula Xa, Xb or Xc wherein M is an aminosulfonyl radical of formula $H_2NS(O_2)-$ with an isocyanate of formula $R^7.NCO$;
(C) for a compound of formula Ia, Ib or Ic wherein A is a radical of formula $R^9.S(O_2).NH.CO.NR^8-$, treating a corresponding compound of formula Xa, Xb or Xc wherein M is an amino radical of formula $HNR^8-$ with a sulfonylisocyanate of formula $R^9.S(O_2).NCO$;
(D) for a compound of formula Ia, Ib or Ic wherein A is a radical of formula $R^9.S(O_2).NH.CO.NR^8-$and wherein $R^8$ is H, treating a corresponding compound of formula Xa, Xb or Xc wherein M is an isocyanate radical of formula ONC- with a sulfonamide of formula $R^9.S(O_2).NH_2$;
(E) for a compound of formula Ia, Ib or Ic wherein A is a radical of formula Q.B- in which -B- is $-S(O_2)NHCO-$, coupling a corresponding acid of formula Xa, Xb or Xc wherein M is COOH with a sulfonamide of formula $Q.S(O_2).NH_2$;
(F) for a compound of formula Ia, Ib or Ic wherein A is a radical of formula Q.B- in which -B- is $-CO.NH.S(O)_2)-$, coupling a corresponding sulfonamide of formula Xa, Xb or Xc wherein M is $H_2N.S(O_2)-$ with a heterocyclic acid of formula Q.COOH; and
(G) for a compound of formula Ia, Ib or Ic wherein A is a radical of formula $CF_3.S(O_2).NH-$, treating a corresponding amine of formula Xa, Xb or Xc wherein M is $H_2N-$ with trifluoromethanesulfonic anhydride;
and wherein the chemical formulae VIIa, VIIb, VIIc, Xa, Xb and Xc are set out hereinbelow and wherein $R^1$, $R^2$, $R^4$-$R^8$, and A have the meanings defined in above;
and whereafter, when a pharmaceutically acceptable salt is required, reacting an acid form of the compound of formula Ia, Ib or Ic with a base affording a physiologically acceptable cation or reacting a suitably basic compound of formula Ia, Ib or Ic with an acid affording a physiologically acceptable anion.

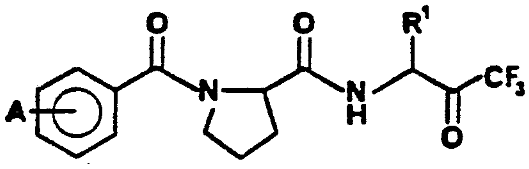

Ia

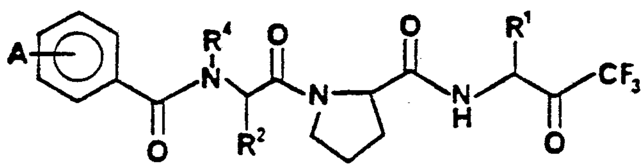

Ib

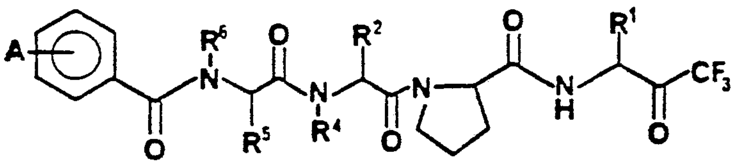

Ic

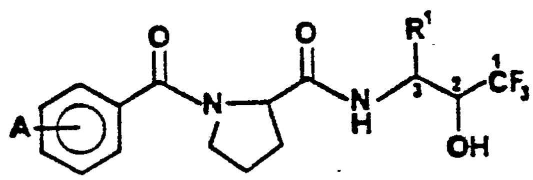

VIIa

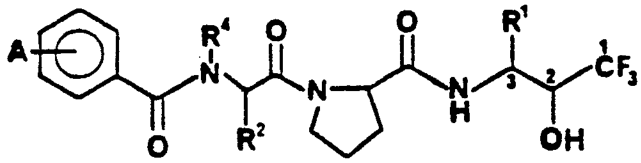

VIIb

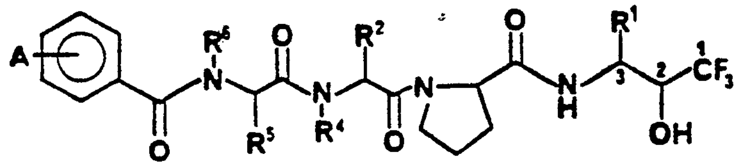

VIIc

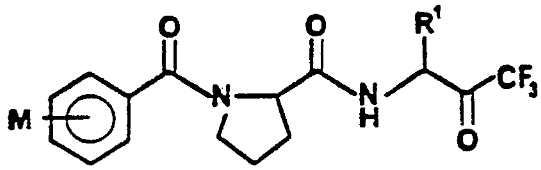

Xa

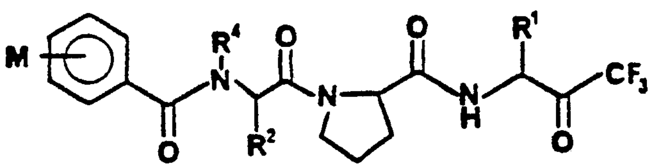

Xb

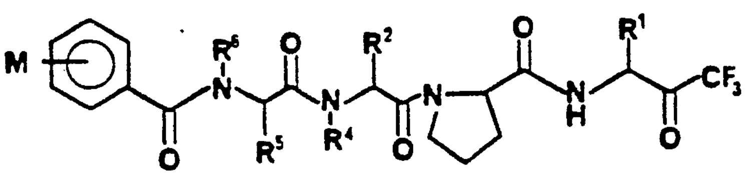

Xc